# EUROPÄISCHE PATENTSCHRIFT

(11) **EP 1 829 613 B1**
(45) Veröffentlichungstag und Bekanntmachung des Hinweises auf die Patenterteilung: **30.03.2022**
(21) Anmeldenummer: 07102614.0
(22) Anmeldetag: 19.02.2007
(51) Int. Cl.: B01L 9/00, A61B 10/00, G01N 35/00, G01N 1/31

(54) **Aufbewahrungseinheit und Transfersystem zum Aufbewahren und Bereitstellen von Biologischen proben**
Storage unit and transfer system for storing and providing biological samples
Unité de stockage et système de transfert destinés au stockage et la préparation d'échantillons biologiques

(30) Priorität: 02.03.2006 CH 3342006
(43) Veröffentlichungstag der Anmeldung: 05.09.2007
(73) Patentinhaber: Nexus Biosystems, Inc., Poway, California 92064 (US)
(72) Erfinder: Elsener, Donat, 3600 Thun (CH); Reisch, Dietmar, 3600 Thun (CH)
(74) Vertreter: Eisenführ Speiser

(56) Entgegenhaltungen:
- EP-A1- 1 621 296
- EP-A2- 0 611 598
- WO-A1-96/30124
- DE-A1- 2 753 223
- DE-U1- 29 712 535
- US-A- 6 004 512
- US-A1- 2001 002 986
- US-A1- 2001 002 986
- US-A1- 2001 003 652
- US-A1- 2003 138 353
- US-A1- 2004 013 576
- US-A1- 2005 232 820

## Beschreibung

Die Erfindung betrifft gemäss dem Oberbegriff des unabhängigen Anspruchs 1 eine Aufbewahrungseinheit für biologische Proben, mit einer horizontalen Hauptstandfläche und mehreren Aufbewahrungskompartimenten, die zumindest teilweise durch Zwischenwände voneinander getrennt und von einem Umfangsrahmen umgeben sind, wobei die Zwischenwände und der Umfangsrahmen rechtwinklig zur Hauptstandfläche angeordnet sind, und wobei diese Aufbewahrungskompartimente sowohl eine erste Öffnung als auch eine zweite Öffnung aufweisen.

Die Erfindung betrifft gemäss dem Oberbegriff des Anspruchs 16 zudem ein Transfersystem, welches eine Vorrichtung umfasst, mit welcher mindestens zwei solche Aufbewahrungseinheiten oder mindestens eine solche Aufbewahrungseinheit und eine Sammeleinheit im Register übereinander angeordnet und gegenüber einander verschoben werden können.

Die Erfindung betrifft des Weiteren gemäss dem Oberbegriff des Anspruchs 22 die Verwendung von in individuellen Aufbewahrungskompartimenten von solchen Aufbewahrungseinheiten für biologische Proben angeordneten Biopsiekassetten oder Glasobjektträgern zum Aufbewahren und Bereitstellen solcher biologischen Proben. Dabei umfasst jede solche Aufbewahrungseinheit eine horizontale Hauptstandfläche und mehrere Aufbewarungskompartimente, die zumindest teilweise durch Zwischenwände voneinander getrennt und von einem Umfangsrahmen umgeben sind. Diese Zwischenwände und der Umfangsrahmen sind rechtwinklig zur Hauptstandfläche angeordnet. Zudem weisen diese Aufbewahrungskompartimente sowohl eine erste Öffnung als auch eine zweite Öffnung auf.

In biologischen Labors, insbesondere in den Labors von pathologischen Instituten von Universitäten oder Spitälern, werden biologische Proben, z.B. durch Biopsie gewonnene Gewebeproben, sehr oft als Gewebestücke in Kassetten oder als Dünnschnitte auf Glasobjektträgern aufbewahrt. Ein Auswahl solcher Kassetten und Glasobjektträger wird z.B. von THERMO SHANDON angeboten. Typischerweise bestehen diese Kassetten aus einem flachen, quaderförmigen Probenkorb mit senkrecht stehenden geschlossenen Seitenwänden und einer gitterförmig durchbrochenen, unteren Lagerfläche. Die obere Lagerfläche wird zumeist durch einen Klappdeckel mit Schnappverschluss bereitgestellt, der über ein Gelenk mit dem Probenkorb verbunden ist und ebenfalls gitterförmig durchbrochen ist. Diese Proben werden standardmässig, beispielsweise in Paraffin eingebettet (vgl. z.B. US 5,665,398, US 5,968,436 oder DE 43 06 310 A1) und bei Raumtemperatur, im Kühlschrank (bei ca. +4 °C), im Tiefkühler (bei ca. -18 °C), bei tiefer Temperatur in der Umgebung von festem CO₂ (bei ca. -80 °C) oder bei extrem tiefer Temperatur in flüssigem Stickstoff (bei ca. -196 °C) aufbewahrt. Eine sehr grosse Anzahl von solchen Proben, die in die Hunderttausende, wenn nicht gar in die Millionen gehen kann, verkompliziert das Auffinden einer bestimmte Probe. Dieses Auffinden wird mit abnehmender Lagertemperatur immer schwieriger. Gerade das gezielte Herausnehmen einer einzelnen Probe aus einem Flüssigstickstoffbehälter ist in der Regel nicht möglich. Normalerweise muss eine Container, der viele Proben enthält, aus dem Stickstofftank gezogen werden, so dass dann die gesuchte Probe ausgewählt werden kann. Dies ist ein zeitraubender Prozess, bei dem zudem die Unversehrtheit und Qualität der anderen, nicht ausgewählten Proben mehr oder weniger aufs Spiel gesetzt wird.

Das gemeinsame Lagern von Proben stellt für die Fragestellungen der Pathologie üblicherweise kein Problem dar, eine Kontamination von Nachbarproben durch ein sogenanntes "Carry Over" kann im Allgemeinen vernachlässigt werden. Eine Auswahl von Behältern zum Lagern und Bereitstellen solcher Kassetten und Glasobjektträger (insbesondere bei Raumtemperatur, vgl. auch EP 1 148 372 A2) wird ebenfalls von THERMO SHANDON angeboten. Ganz andere Anforderungen stellen in diesem Zusammenhang Labors von Universitäten und der Pharmaindustrie, die sich mit Proteinstudien an oder in gefrorenen Proben beschäftigen.

In der Pharmaforschung werden chemische oder biochemische Verbindungen routinemässig auf ihre potenzielle pharmazeutische Aktivität geprüft. Zu diesem Zweck muss eine grosse Anzahl von Proben innert kürzester Zeit bereitgestellt werden. In Laboratorien der Pharmaforschung werden deshalb sogenannte Mikroröhrchen ("Micro-Tubes") verwendet, welche eine genügende Menge einer bestimmten Substanz enthalten. Um möglichst ökonomisch mit den riesigen Zahlen solcher Mikroröhrchen umgehen zu können, werden diese in sogenannte "Micro-Tube Cluster Racks" gepackt. Dabei sind zum robotisierten Handling solche Racks speziell bevorzugt, welche eine Standfläche aufweisen, die dem sogenannten "Foot Print" einer Mikroplatte nach dem SBS-Standard (SBS = Society for Biomoiecular Screening) entspricht und deshalb oft als "SBS footprint" bezeichnet wird. Inzwischen wurde dieser Standard vom ANSI (American National Standards Institute) als ANSI/SBS 1-2004 normiert. Micro-Tube Cluster Racks mit 96 oder 384 Mikro-Röhrchen sind beispielsweise unter dem Handlesnahmen REMP Tube Technology^{™} bekannt.

Dünnschnitte von fixierten, beispielsweise in Paraffin eingebetteten Proben werden in der Pathologie routinemässig auf Glasobjektträgern aufgetragen und mittels Lichtmikroskopie beurteilt. Die folgende Tabelle 1 gibt einen Überblick über die gebräuchlichsten Glasobjektträger:

**Tabelle 1:**

| Typ | | Inch: 1 x 3 Zoll | Metrisch: 25 x 75 mm |
|---|---|---|---|
| | | | |
| Dimensionen | Länge x Breite (Toleranzen) | 76.2 mm x 25.4 mm (± 0.5 mm) | 75 mm x 25 mm (± 0.5 mm) |
| | | | |
| Dicke | "standard" | 1.02 mm (± 0.05 mm) | 1.02 mm (± 0.05 mm) |
| | "dick" | 1.2 mm (± 0.1 mm) | |
| | | | |
| Behandlung | Ecken | scharf, gefast | scharf, gerundet |
| | Kanten | scharf, gefast | scharf |
| | Oberflächen | blank, sandgestrahlt, bemalt auf einer oder beiden Seiten | blank, sandgestrahlt, bemalt auf einer oder beiden Seiten |

| | | | |
|---|---|---|---|
| (gemäss: Schermer, M.J.: Confocal scanning microscopy in microarray detection; in "DNA Microarrays, A practical approach"; Mark Schena (ed.), Oxford University Press 1999, 17-42) | | | |

Der aktuelle Anmelder selbst vertreibt Micro-Tube Cluster Racks mit 96 oder 384 Mikro-Röhrchen unter dem Handelsnamen REMP Tube Technology^{™}. Diese unterscheiden sich im Wesentlichen dadurch von den Racks und Mikro-Röhrchen aus dem übrigen Stand der Technik, dass das Bereitstellen der Proben-Röhrchen erfolgt, indem zumindest zwei Racks übereinander angeordnet werden und Probenröhrchen mit einem Manipulator aus dem obenliegenden Rack in entsprechend positionierte Aufnahmekavitäten des untenliegenden Racks gestossen werden. Umgekehrt kann dieser Übergabeprozess aber auch dadurch erfolgen, dass Probenröhrchen mit einem Manipulator aus dem untenliegenden Rack in entsprechend positionierte Aufnahmekavitäten des obenliegenden Racks gestossen werden (vgl. z.B. EP 0 904 841 B1 oder US 6,827,907 B2).

US 2001/003652 A1 offenbart ein Trägerhalteelement, welches so beschaffen ist, dass es zusammen mit einem Träger eine Trägerzelle bildet, wobei die Trägerzelle eine im Wesentlichen abgedichtete Kammer umfasst, wobei die Kammer mit einem Fluideinlass versehen ist und einen Fluidauslass zum Einführen bzw. Entfernen von Fluiden, die bei der Verarbeitung der Probe verwendet werden.

US 2003/0138353 A1 offenbart eine Kartusche zum Bearbeiten einer Probe, mit einem Trägerelement, einem Gehäuse mit einem Hohlraum mit einer Öffnung auf, durch die das Trägerelement in den Hohlraum eingeführt werden kann.

Die Aufgabe der vorliegenden Erfindung besteht darin, eine - in Bezug auf die eingangs erwähnte - alternative Aufbewahrungseinheit zum Aufbewahren und Bereitstellen einer grossen Anzahl von biologischen Proben vorzuschlagen.

Diese Aufgabe wird durch die Merkmale des unabhängigen Anspruchs 1 erfüllt, indem eine Aufbewahrungseinheit für biologische Proben mit einer horizontalen Hauptstandfläche und mehreren Aufbewahrungskompartimenten vorgeschlagen wird. Diese Aufbewahrungskompartimente sind zumindest teilweise durch Zwischenwände voneinander getrennt und von einem Umfangsrahmen umgeben. Dabei sind die Zwischenwände und der Umfangsrahmen rechtwinklig zur Hauptstandfläche angeordnet. Zudem weisen diese Aufbewahrungskompartimente sowohl eine erste Öffnung als auch eine zweite Öffnung auf. Die Aufbewahrungskompartimente umfassen Haltemittel. Je eine Biopsiekassette oder je ein Glasobjektträger ist gegen einen Gleitwiderstand der Haltemittel durch die erste Öffnung und durch die zweite Öffnung in ein solches Aufbewahrungskompartiment einsetzbar. Die erfindungsgemässe Aufbewahrungseinheit für biologische Proben ist dadurch gekennzeichnet, dass die Aufbewahrungskompartimente an die Form einer Biopsiekassette oder an die Form eines Glasobjektträgers angepasst sind und zumindest eines der Aufbewahrungskompartimente eine solche Biopsiekassette oder einen solchen Glasobjektträger umfasst und die Haltemittel dazu angepasst sind, die vertikal stehenden Biopsiekassetten oder Glasobjektträger am Herausfallen durch die erste Öffnung und am Herausfallen durch die zweite Öffnung zu hindern, wobei die Haltemittel so ausgebildet sind, dass die erste Öffnung und die zweite Öffnung offengehalten werden. Zusätzliche bevorzugte und erfinderische Merkmale ergeben sich aus den abhängigen Ansprüchen.

Vorteile der erfindungsgemässe Aufbewahrungseinheit für biologische Proben umfassen:
- Die Biopsiekassetten oder Glasobjektträger mit den biologischen Proben können robotisiert in die Aufbewahrungskompartimente eingesetzt werden.
- Die Biopsiekassetten oder Glasobjektträger mit den biologischen Proben können robotisiert aus den Aufbewahrungskompartimenten entnommen werden.
- Die Biopsiekassetten oder Glasobjektträger mit den biologischen Proben können robotisiert von einem Aufbewahrungskompartiment in ein anderes Aufbewahrungskompartiment der selben Aufbewahrungseinheit umplatziert werden.
- Die Biopsiekassetten oder Glasobjektträger mit den biologischen Proben können robotisiert von einem Aufbewahrungskompartiment in ein anderes Aufbewahrungskompartiment einer anderen Aufbewahrungseinheit umplatziert werden.
- Jede Biopsiekassette und jeder Glasobjektträger mit den darin oder darauf enthaltenen biologischen Proben kann individuell adressiert und ohne jede Beeinflussung anderer Proben robotisiert gelagert und bereitgestellt werden.
- Das robotisierte Lagern und Bereitstellen der Biopsiekassetten und Glasobjektträger mit den darin oder darauf enthaltenen biologischen Proben kann praktisch bei beliebigen Temperaturen erfolgen.

Beispielhafte Ausführungsformen der erfindungsgemässen Aufbewahrungseinheit werden nun an Hand von schematischen Zeichnungen näher erläutert. Dabei zeigen:
- Fig. 1: eine Draufsicht auf eine für Biopsiekassetten geeignete Aufbewahrungseinheit, in welcher die im wesentlichen vertikal stehenden Biopsiekassetten eng gepackt und quer angeordnet sind;
- Fig. 2: eine Draufsicht auf eine für Biopsiekassetten geeignete Aufbewahrungseinheit, in welcher die im wesentlichen vertikal stehenden Biopsiekassetten eng gepackt und längs angeordnet sind;
- Fig. 3: eine erste Draufsicht auf eine für Biopsiekassetten geeignete Aufbewahrungseinheit, in welcher die im wesentlichen vertikal stehenden Biopsiekassetten locker gepackt und quer angeordnet sind;
- Fig. 4: eine zweite Draufsicht auf eine für Biopsiekassetten geeignete Aufbewahrungseinheit, in welcher die im wesentlichen vertikal stehenden Biopsiekassetten locker gepackt und quer angeordnet sind;
- Fig. 5: Ansichten einer geschlossenen, an sich aus dem Stand der Technik bekannten Biopsiekassette, wobei

- Fig. 5A: eine Draufsicht auf die Lagerfläche des Deckels der Biopsiekassette;
- Fig. 5B: eine Ansicht auf eine Höhenfläche des Korbes der Biopsiekassette; und
- Fig. 5C: eine Ansicht auf die vordere Stirnfläche des Korbes der Biopsiekassette zeigt;

- Fig. 6: einen Vertikalschnitt durch zwei übereinander im Register angeordnete Aufbewahrungseinheiten mit der SBS Norm-Standfläche einer Standard-Mikroplatte, wobei eine Biopsiekassette vertikal von der oberen Aufbewahrungseinheit in die untere gestossen wird;
- Fig. 7: - die ein Beispiel, das das Verständnis der Erfindung erleichtert, isteinen Vertikalschnitt durch zwei übereinander im Register angeordnete Aufbewahrungseinheiten, wobei eine Biopsiekassette horizontal von der unteren Aufbewahrungseinheit in die obere gestossen wird;
- Fig. 8: - deren rechte Seite ein Beispiel, das das Verständnis der Erfindung erleichtert, ist - eine Draufsicht auf eine für Glasobjektträger geeignete Aufbewahrungseinheit, in welcher die Glasobjektträger quer und im wesentlichen vertikal stehend, eng gepackt oder quer und liegend angeordnet sind;
- Fig. 9: einen Vertikalschnitt durch drei übereinander im Register angeordnete Aufbewahrungseinheiten mit der SBS Norm-Standfläche einer Standard-Mikroplatte, wobei je ein stehender Glasobjektträger vertikal von der oberen Aufbewahrungseinheit in die mittlere und von der unteren Aufbewahrungseinheit in die mittlere gestossen wird;
- Fig. 10: - die ein Beispiel, das das Verständnis der Erfindung erleichtert, isteinen Vertikalschnitt durch drei übereinander im Register angeordnete Aufbewahrungseinheiten mit der SBS Norm-Standfläche einer Standard-Mikroplatte, wobei je ein liegender Glasobjektträger vertikal von der oberen Aufbewahrungseinheit in die mittlere und von der unteren Aufbewahrungseinheit in die mittlere gestossen wird;

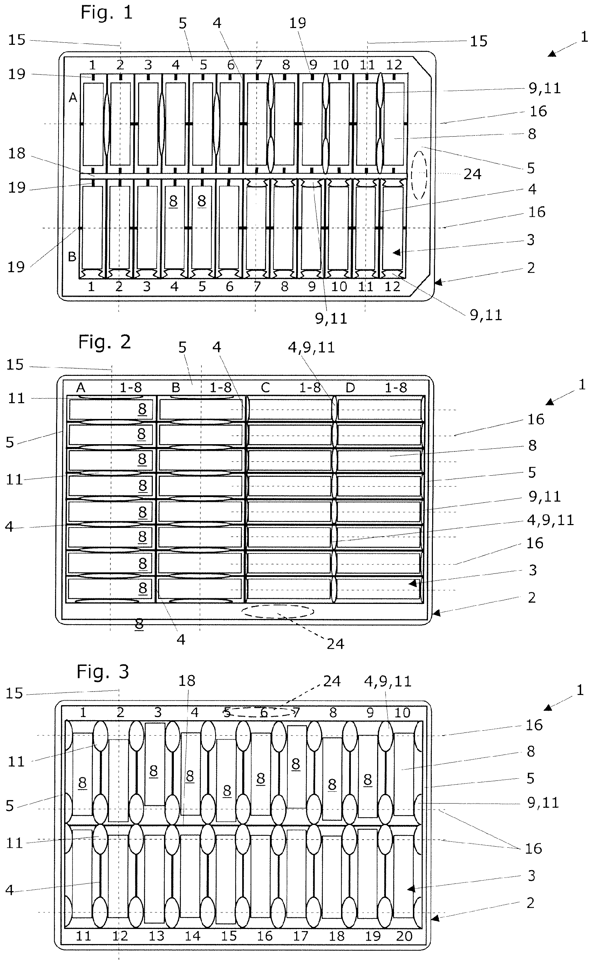

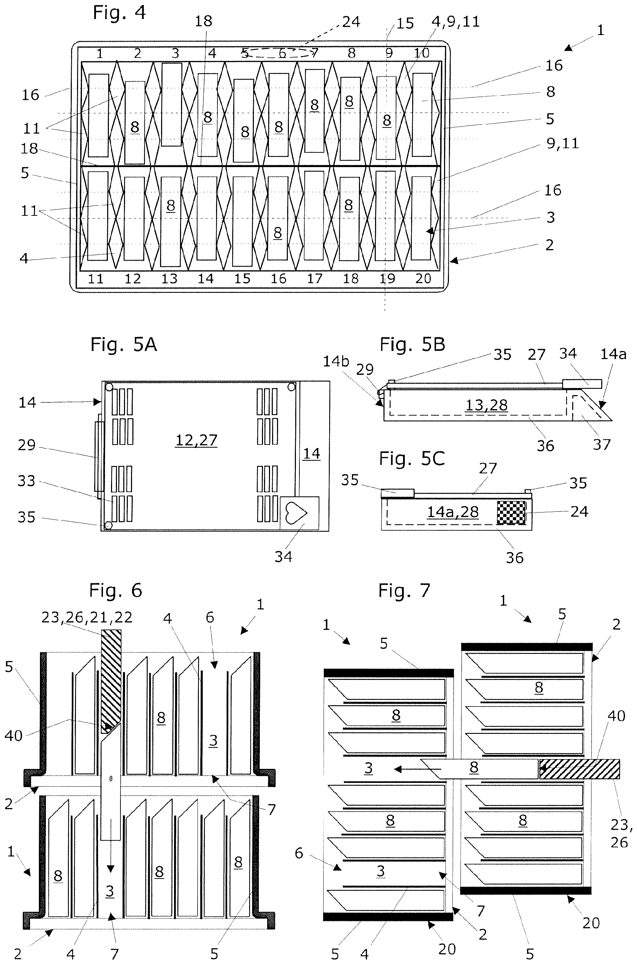

Figur 1 zeigt eine Aufbewahrungseinheit 1 für biologische Proben, mit einer Hauptstandfläche 2 und mehreren Aufbewahrungskompartimenten 3, die zumindest teilweise durch Zwischenwände 4 voneinander getrennt und von einem Umfangsrahmen 5 umgeben sind, wobei die Zwischenwände 4 und der Umfangsrahmen 5 im wesentlichen rechtwinklig zur Hauptstandfläche 2 angeordnet sind, und wobei diese Aufbewahrungskompartimente 3 sowohl eine erste Öffnung 6 als auch eine zweite Öffnung 7 (vgl. Fig. 6 und 7) aufweisen. Die Aufbewahrungskompartimente weisen hier über ihre ganze Höhe in der Aufbewahrungseinheit 1 das gleiche, im wesentlichen rechteckige Querschnittsprofil auf. Die Aufbewahrungskompartimente 3 sind an die Form einer Biopsiekassette 8 angepasst, welche entweder durch die erste Öffnung 6 oder durch die zweite Öffnung 7 in diese Aufbewahrungskompartimente 3 im wesentlichen vertikal stehend eingesetzt worden sind und auf einer ihrer Stirnflächen 14 (vgl. Fig. 5C) stehen. Die Aufbewahrungskompartimente 3 umfassen zudem Haltemittel 9, die hier als einseitige und doppelseitige Kissen 11 ausgebildet sind. Die Haltemittel 9 verlaufen im wesentlichen rechtwinklig in Bezug auf die Hauptstandfläche 2 und sind zur Ineingriffnahme mit Reibschluss an den Lagerflächen 12 (vgl. Fig. 5A) oder an den Höhenflächen 13 (vgl. Fig. 5B) einer in ein Aufbewahrungskompartiment 3 eingesetzten Biopsiekassette 8 ausgebildet. Durch diesen Reibschluss werden diese im wesentlichen vertikal eingesetzten Biopsiekassetten 8 am Herausfallen durch die erste Öffnung 6 und/oder durch die zweite Öffnung 7 (egal, auf welche Seite die Aufbewahrungseinheit 1 gekippt wird) gehindert.

Gemäss einer ersten Ausführungsform sind die Kissen 11 doppelseitig ausgebildet, wie dies in den Aufbewahrungskompartimenten A1 bis A12 gezeichnet ist. Dieses "doppelseitig" soll im Zusammenhang mit der vorliegenden Erfindung als "in zwei Richtungen gegen die anliegenden Biopsiekassetten wirkend" verstanden werden. Diese Kissen 11 sind zur Ineingriffnahme mit Reibschluss an den Lagerflächen 12 einer in ein Aufbewahrungskompartiment 3 eingesetzten Biopsiekassette 8 ausgebildet und auf einer mittig und quer zu diesen Aufbewahrungskompartimenten 3 verlaufenden Längsachse 16 (vgl. Aufbewahrungskompartimente A1 bis A6) angeordnet. Die doppelseitigen Kissen 11 sind einer Reihe Aufbewahrungskompartimenten (vgl. A7 bis A12) auf zwei quer zu diesen Aufbewahrungskompartimenten 3 verlaufenden Längsachsen 16 angeordnet. Um die Stabilität der Aufbewahrungseinheit 1 zu verbessern, weist diese vorzugsweise eine Mittelwand 18 auf. Den einzusetzenden Biopsiekassetten 8 wird durch Führungslamellen 19 eine zusätzliche Führung gegeben. Diese Führungslamellen 19 verlaufen vorzugsweise rechtwinklig in Bezug auf die Hauptstandfläche 2 und können wahlweise an der Mittelwand 18, an den Zwischenwänden 4 und an dem Umfassungsrahmen 5 angeordnet werden. Diese Führungslamellen 19 stehen vorzugsweise rechtwinklig von ihrer tragenden Wand in die Aufbewahrungskompartimente 3 ab; speziell bevorzugt sind solche Führungslamellen 19, die sich gegen die erste und zweite Öffnung 6,7 verjüngen und so einen Einlauf für die einzusetzenden Biopsiekassetten 8 bilden (nicht gezeigt).

Gemäss einer zweiten Ausführungsform sind die Kissen 11 einseitig ausgebildet, wie dies in den Aufbewahrungskompartimenten B1 bis B12 gezeichnet ist. Dieses "einseitig" soll im Zusammenhang mit der vorliegenden Erfindung als "in einer Richtung gegen die anliegende Biopsiekassette wirkend" verstanden werden. Diese Kissen 11 sind zur Ineingriffnahme mit Reibschluss an den Höhenflächen 13 einer in ein Aufbewahrungskompartiment 3 eingesetzten Biopsiekassette 8 ausgebildet und auf einer mittig und längs zu diesen Aufbewahrungskompartimenten 3 verlaufenden Querachse 15 angeordnet.

Wie bereits an Hand von Figur 1 gezeigt wurde, lassen sich die Elemente Kissen 11 und Führungslamellen 19 praktisch beliebig auswählen bzw. untereinander kombinieren. Bevorzugt entspricht die Hauptstandfläche 2 einer solchen Aufbewahrungseinheit 1 zumindest annähernd der SBS Norm-Standfläche einer Standard-Mikroplatte.

Mit dem Ziel, möglichst viele Biopsiekassetten 8 in der in Fig. 1 gezeigten Aufbewahrungseinheit 1 unterzubringen, wurden zwei Reihen mit je zwölf Aufbewahrungskompartimenten 3 im wesentlichen vertikal stehend so angeordnet, dass die in Bezug auf die Hauptstandfläche 2 auf ihren Stirnflächen 14 stehenden Biopsiekassetten 8 quer zur Längsrichtung der Aufbewahrungseinheit 1 angeordnet sind. Mit den gewählten Achsabständen von etwa 9 mm ergibt dies ein Total von 24 Kassetten 8 pro Aufbewahrungseinheit 1. Würde man den Umfangsrahmen 5 verschmälern, so könnten zwei zusätzliche Kassetten in dieser Aufbewahrungseinheit 1 untergebracht werden; allerdings würden dann die Positionsbezeichnungen wesentlich kleiner ausfallen oder diese müssten ganz weggelassen werden.

Wie gezeigt, weist die Aufbewahrungseinheit 1 zwei abgeschrägte Ecken als Orientierungshilfen auf. Auf diese Orientierungshilfen könnte teilweise oder ganz verzichtet werden. Alternative Orientierungshilfen können beispielsweise mit einer abgerundeten Ecke oder auch mit einer mit einer Kerbe oder einem speziellen Relief ausgestatteten Ecke (nicht gezeigt) bereitgestellt werden. Eine weitere Möglichkeit bietet das Anbringen eines Barcodes auf einer Aussenfläche des Umfangsrahmens und/oder das Anbringen einer Radiofrequenz-Etikette, d.h. eines sogenannten "RF-ID Tags". Speziell bevorzugt sind RF-ID Tags, die nicht nur einen individuellen, gespeicherten und abrufbaren Identifikationscode aufweisen, sondern die zudem einen beschreibbaren und abrufbaren zusätzlichen Datenspeicher umfassen. Das Anbringen dieser RF-ID Tags an Orten an oder in der Aufbewahrungseinheit 1, an denen keine mechanische Einwirkung auf diese RF-ID Tags befürchtet werden muss, ist bevorzugt. Barcodes werden alternativ oder ergänzend zu den RF-ID Tags verwendet liegen vorzugsweise in eindimensionaler oder zweidimensionaler Form vor.

Figur 2 zeigt eine Draufsicht auf eine für Biopsiekassetten geeignete Aufbewahrungseinheit 1 biologische Proben, in welcher die im wesentlichen vertikal stehenden Biopsiekassetten 8 eng gepackt und längs in Bezug auf die Längsausrichtung der Aufbewahrungseinheit 1 angeordnet sind. Auch diese Aufbewahrungseinheit 1 umfasst eine Hauptstandfläche 2 und mehrere Aufbewahrungskompartimente 3, die zumindest teilweise durch Zwischenwände 4 voneinander getrennt und von einem Umfangsrahmen 5 umgeben sind. Dabei sind die Zwischenwände 4 und der Umfangsrahmen 5 im wesentlichen rechtwinklig zur Hauptstandfläche 2 angeordnet. Zudem weisen diese Aufbewahrungskompartimente 3 sowohl eine erste Öffnung 6 als auch eine zweite Öffnung 7 (vgl. Fig. 6 und 7) auf. Die Aufbewahrungskompartimente 3 weisen hier über ihre ganze Höhe in der Aufbewahrungseinheit 1 das gleiche, im wesentlichen rechteckige Querschnittsprofil auf. Die Aufbewahrungskompartimente 3 sind an die Form einer Biopsiekassette 8 angepasst, welche entweder durch die erste Öffnung 6 oder durch die zweite Öffnung 7 in diese Aufbewahrungskompartimente 3 eingesetzt worden sind und auf einer ihrer Stirnflächen 14 (vgl. Fig. 5C) stehen. Die Aufbewahrungskompartimente 3 umfassen zudem Haltemittel 9, die hier als einseitige und doppelseitige Kissen 11 ausgebildet sind. Die Haltemittel 9 verlaufen im wesentlichen rechtwinklig in Bezug auf die Hauptstandfläche 2 und sind zur Ineingriffnahme mit Reibschluss an den Lagerflächen 12 (vgl. Fig. 5A) oder an den Höhenflächen 13 (vgl. Fig. 5B) einer in ein Aufbewahrungskompartiment 3 eingesetzten Biopsiekassette 8 ausgebildet. Durch diesen Reibschluss werden diese eingesetzten Biopsiekassetten 8 am Herausfallen durch die erste Öffnung 6 und/oder durch die zweite Öffnung 7 (egal, auf weiche Seite die Aufbewahrungseinheit 1 gekippt wird) hindern. Auch hier sind die Kissen 11 einseitig oder doppelseitig ausgebildet.

In den Aufbewahrungskompartimenten A1, A8, B1 und B8 sind die Kissen 11 am Umfassungsrahmen 5 angeordnet und einseitig wirkend ausgebildet. In den Aufbewahrungskompartimenten A2 bis A7 und B2 bis B7 sind die Kissen 11 an den Zwischenwänden 4 angeordnet und doppelseitig wirkend ausgebildet. In den Aufbewahrungskompartimenten C1 bis C8 und D1 bis D8 sind die Kissen 11 am Umfassungsrahmen 5 oder an den Zwischenwänden 4 angeordnet und entsprechend einseitig oder doppelseitig wirkend ausgebildet. Alle diese Kissen 11 sind zur Ineingriffnahme mit Reibschluss an den Lagerflächen 12 oder an den Höhenflächen 13 einer in ein Aufbewahrungskompartiment 3 eingesetzten Biopsiekassette 8 ausgebildet. Die Hälfte davon (vgl. Aufbewahrungskompartimente A1 bis A8 und B1 bis B8) sind auf einer mittig und quer zu diesen Aufbewahrungskompartimenten 3 verlaufenden Querachse 15 angeordnet. Die andere Hälfte (vgl. Aufbewahrungskompartimente C1 bis C8 und D1 bis D8) sind auf einer mittig und längs zu diesen Aufbewahrungskompartimenten 3 verlaufenden Längsachse 16 angeordnet.

Wie gezeigt können die ganzen oder ein Teil der Mittelwände durch die Kissen 11 gebildet werden. Um die Stabilität der Aufbewahrungseinheit 1 zu verbessern, kann diese eine verstärkte Mittelwand 18 aufweisen (nicht gezeigt). Wegen den hier herrschenden, engen Platzverhältnissen, die eine recht genaue Positionierung der Biopsiekassetten 8 bewirken, wurde hier auf Führungslamellen 19 verzichtet. Mit dem Ziel, möglichst viele Biopsiekassetten 8 in der in Fig. 2 gezeigten Aufbewahrungseinheit 1 unterzubringen, wurden vier Reihen mit je acht Aufbewahrungskompartimenten 3 so angeordnet, dass die in Bezug auf die Hauptstandfläche 2 auf ihren Stirnflächen 14 stehenden Biopsiekassetten 8 längs zur Längsrichtung der Aufbewahrungseinheit 1 angeordnet sind. Mit den gewählten Achsabständen von etwa 9 mm ergibt dies ein Total von 32 Kassetten 8 pro Aufbewahrungseinheit 1. Würde man den Umfangsrahmen 5 verschmälern, so könnten vier zusätzliche Kassetten in dieser Aufbewahrungseinheit 1 untergebracht werden; allerdings würden dann die Positionsbezeichnungen wesentlich kleiner ausfallen oder diese müssten ganz weggelassen werden.

Figur 3 zeigt eine erste Draufsicht auf eine für Biopsiekassetten geeignete Aufbewahrungseinheit 1, in welcher die im wesentlichen vertikal stehenden Biopsiekassetten 8 locker gepackt und quer angeordnet sind. Im Gegensatz zu den beiden vorgehend beschriebenen Aufbewahrungseinheiten 1, wurde hier die Anzahl der einsetzbaren Biopsiekassetten 8 auf total zwanzig reduziert und es wurden Kissen 11 mit einem grösseren Volumen verwendet. Zudem wurde auf Führungslamellen 19 verzichtet. Auch hier umfasst die Aufbewahrungseinheit 1 eine Hauptstandfläche 2 und mehrere Aufbewahrungskompartimente 3, die zumindest teilweise durch Zwischenwände 4 voneinander getrennt und von einem Umfangsrahmen 5 umgeben sind. Die Zwischenwände 4 und der Umfangsrahmen 5 sind ebenfalls im wesentlichen rechtwinklig zur Hauptstandfläche 2 angeordnet. Zudem weisen diese Aufbewahrungskompartimente 3 sowohl eine erste Öffnung 6 als auch eine zweite Öffnung 7 (vgl. Fig. 6 und 7) auf. Die Aufbewahrungskompartimente 3 weisen hier über ihre ganze Höhe in der Aufbewahrungseinheit 1 das gleiche, im wesentlichen rechteckige Querschnittsprofil auf. Die Aufbewahrungskompartimente 3 sind an die Form einer Biopsiekassette 8 angepasst, welche entweder durch die erste Öffnung 6 oder durch die zweite Öffnung 7 in diese Aufbewahrungskompartimente 3 eingesetzt worden sind und auf einer ihrer Stirnflächen 14 (vgl. Fig. 5C) stehen. Zweck dieser Ausführungsform ist es einerseits, solche Aufbewahrungskompartimente 3 bereitzustellen, welche den Dimensionen der einzusetzenden Biopsiekassetten 8 einen grösseren Spielraum lassen. Andererseits ermöglicht diese Ausführungsform der Aufbewahrungseinheit 1 ein recht ungenaues Einsetzen der Biopsiekassetten 8, stellt aber trotzdem das sichere Halten dieser Biopsiekassetten 8 in den Aufbewahrungskompartimenten 3 sicher. Um die Stabilität der Aufbewahrungseinheit 1 zu verbessern, kann diese eine verstärkte Mittelwand 18 aufweisen (nicht gezeigt).

In den Aufbewahrungskompartimenten 1, 10, 11 und 20 sind die einseitig wirkenden Kissen 11 am Umfassungsrahmen 5 angeordnet. Alle doppelseitig wirkenden Kissen 11 sind im Bereich der Zwischenwände angeordnet. Alle diese Kissen 11 sind zur Ineingriffnahme mit Reibschluss an den Lagerflächen 12 einer in ein Aufbewahrungskompartiment 3 eingesetzten Biopsiekassette 8 ausgebildet und in einer Reihe Aufbewahrungskompartimenten (vgl. 1 bis 10 und 11 bis 20) auf zwei quer zu diesen Aufbewahrungskompartimenten 3 verlaufenden Längsachsen 16 angeordnet.

Figur 4 zeigt eine zweite Draufsicht auf eine für Biopsiekassetten geeignete Aufbewahrungseinheit 1, in welcher die im wesentlichen vertikal stehenden Biopsiekassetten 8 locker gepackt und quer angeordnet sind. Im Unterschied zu Figur 3 sind die Kissen 11 in ihrem Querschnitt rhomboid und nicht elliptisch ausgeführt. Zudem wurde die erhabensten Punkte der Kissen so gelegt, dass sie in einem Abstand von etwa einem Viertel der Länge eines Aufbewahrungskompartiments 3 von dem Umgebungsrahmen 5 oder der Mittelwand 18 entfernt liegen. Diese Anordnung ermöglicht es wiederum, Aufbewahrungskompartimente 3 bereitzustellen, welche den Dimensionen der einzusetzenden Biopsiekassetten 8 einen grösseren Spielraum lassen. Andererseits ermöglicht auch diese Ausführungsform der Aufbewahrungseinheit 1 ein recht ungenaues Einsetzen der Biopsiekassetten 8, stellt aber trotzdem das sichere Halten dieser Biopsiekassetten 8 in den Aufbewahrungskompartimenten 3 sicher. Um die Stabilität der Aufbewahrungseinheit 1 zu verbessern, kann diese eine verstärkte Mittelwand 18 aufweisen (nicht gezeigt). Die Zwischenwände 4 sind hier ganz durch die Kissen 11 ersetzt, was einen grösseren Federweg für die Kissen bereitstellt.

Weil die Ausführungsformen der Aufbewahrungskompartimente 3 gemäss Figur 3 und Figur 4 einen grösseren Raum um die eingesetzten Biopsiekassetten 8 einschliessen, eignen sich derartige Aufbewahrungseinheiten 1 speziell gut für die Lagerung der Biopsiekassetten 8 bei tiefen (-80 °C) oder bei sehr tiefen Temperaturen (-196 °C). Dieser grössere Raum verbessert den Gasaustausch in der unmittelbaren Umgebung einer Kassette, so dass diese durch das CO₂-Kühlgas oder den Flüssigstickstoff gut erreichbar ist und optimal gekühlt wird. Die einseitigen oder die doppelseitigen Kissen 11 erstrecken sich vorzugsweise mit gleichem Wirkungsquerschnitt über im wesentlichen die gesamte Höhe der Aufbewahrungskompartimente 3 und sind bevorzugt im Bereich der ersten Öffnung 6 und der zweiten Öffnung 7 offen ausgebildet. Diese Kissen können zudem Durchbrüche aufweisen oder netzartig ausgebildet sein (nicht gezeigt).

Die Figur 5 zeigt Ansichten einer geschlossenen, an sich aus dem Stand der Technik bekannten Biopsiekassette aus spritzgegossenem Kunststoff, wie z.B. Polypropylen. Der Innenraum einer solchen Kassette misst üblicherweise ca. 30 x 25 x 5 mm. Die Figur zeigt 5A eine Draufsicht auf die Lagerfläche 12 des Deckels 27 der Biopsiekassette 8. Der Deckel 27 weist eine Vielzahl von Schlitzen 33 auf, von denen nur ein Teil gezeichnet ist. Der Deckel 27 ist über das Scharnier 29 mit dem Korb 28 verbunden, von dem auf der rechten Seite die abgeschrägte vordere Stirnfläche 14a gut sichtbar ist. Eine am Deckel angeformte Lasche 34 erleichtert das Öffnen der Kassette von Hand. In den von der Lasche nicht besetzten Ecken der Biopsiekassette 8 befinden sich Zapfen 35, welche zusammen mit der Lasche 34 ein gemeinsames Niveau definieren.

Figur 5B zeigt eine Ansicht auf eine Höhenfläche 13 des Korbes 28 der Biopsiekassette 8. Auf der linken Seite sind das Scharnier 29 und ein Zapfen 35 zu sehen. Auf der rechten Seite sind die Lasche 34 und die abgeschrägte vordere Stirnfläche 14a bezeichnet. Der Verlauf des Bodens 36 der Kassette ist gestrichelt eingetragen.

Figur 5C zeigt eine Ansicht auf die abgeschrägte vordere Stirnfläche 14a des Korbes 28 der Biopsiekassette 8. Im Bereich des Deckels 27 sind die Lasche 34 und ein Zapfen 35 sichtbar. Auf der rechten Seite ist ein zweidimensionaler Barcode aufgedruckt. Der Verlauf des Bodens 36, der ein ähnliches Schlitzmuster wie der Deckel 27 der Kassette umfasst (nicht gezeigt) ist gestrichelt eingetragen.

Figur 6 zeigt einen Vertikalschnitt durch zwei übereinander im Register angeordnete Aufbewahrungseinheiten 1 mit einer der SBS Norm-Standfläche einer Standard-Mikroplatte entsprechenden Hauptstandfläche 2, wobei gerade in diesem Moment eine Biopsiekassette 8 von einem Manipulator 23 zumindest im wesentlichen vertikal von der oberen Aufbewahrungseinheit in die untere (gegen die Hauptstandfläche 2 hin) gestossen wird. Der Manipulator 23 ist Teil der Vorrichtung 22 eines Transfersystems 21, mit welchem mindestens zwei Aufbewahrungseinheiten 1 im Register übereinander angeordnet und gegenüber einander verschoben werden können. Die gezeigten Aufbewahrungseinheiten 1 befinden sich in einer gegenseitigen Position, bei welcher alle einander entsprechenden Aufbewahrungskompartimente 3 dieser beiden Aufbewahrungseinheiten 1 genau über einander platziert sind. Zumindest eine dieser beiden Aufbewahrungseinheiten 1 kann nun gegenüber der anderen unter Verwendung der Vorrichtung 22 so verschoben werden, dass jedem beliebigen Aufbewahrungskompartiment 3 der oberen Aufbewahrungseinheit 1 ein beliebiges Aufbewahrungskompartiment 3 der unteren Aufbewahrungseinheit 1 exakt im Register untereinander stehend zugeordnet werden kann. Es ist dann ein Leichtes, einen vorzugsweise robotisierten Manipulator 23 in ein bestimmtes und individuell ausgewähltes Aufbewahrungskompartiment 3 einzuführen und die sich darin befindende Biopsiekassette 8 in ein vorher zugeordnetes Aufbewahrungskompartiment 3 einer zweiten Aufbewahrungseinheit 1 (gegen die Hauptstandfläche 2 hin) zu stossen. Der durch den Reibschluss bewirkte Gleitwiderstand zwischen den Haltemitteln 9 und der zu verschiebenden Biopsiekassette 8 bewirkt, dass diese Biopsiekassette nach dem Übertragen aus der obenliegenden Aufbewahrungseinheit 1 in die untenliegende Aufbewahrungseinheit 1 nicht aus dieser herausfällt. Der Manipulator 23 ist vorzugsweise an die Form der vorderen, abgeschrägten Stirnfläche 14a der Biopsiekassette 8 angepasst und kann eine reibungsverstärkende Beschichtung aufweisen, welche das Abrutschen auf der abgeschrägten Stirnfläche 14a der Biopsiekassette 8 verhindert.

Wie dargestellt, werden die Biopsiekassetten 8 vorzugsweise so im wesentlichen vertikal in die Aufbewahrungskompartimente 3 einer Aufbewahrungseinheit 1 eingesetzt, dass die vorderen, abgeschrägten Stirnflächen 14a der Biopsiekassetten 8 nach oben stehen und aus dieser Richtung von Auge oder automatisiert mittels eines entsprechenden Lesegerätes abgelesen werden können. Ein RF-ID Tag wird vorzugsweise im Blindraum 37 an der Unterseite der Biopsiekassette 8 untergebracht (vgl. Fig. 5B).

Alternativ zu der gezeigten Stossrichtung des Manipulators 23 kann diese auch in der Gegenrichtung, also von unten nach oben verlaufen (nicht gezeigt). Von der Darstellung in Fig. 6 abweichend kann die untenliegende Aufbewahrungseinheit einen geschlossenen Boden aufweisen (nicht gezeigt) und lediglich als Sammeleinheit für Biopsiekassetten 8 dienen. Somit genügt zum Durchführen des alternativen Verfahrens, bei dem ausgewählte Biopsiekassetten 8 von einer Aufbewahrungseinheit 1 oder mehreren solchen Aufbewahrungseinheiten auf eine Sammeleinheit für Biopsiekassetten 8 übertragen werden, die Verwendung von mindestens einer Aufbewahrungseinheit 1 für Biopsiekassetten 8.

Figur 7 zeigt einen Vertikalschnitt durch zwei übereinander im Register angeordnete Aufbewahrungseinheiten 1 mit einer von der SBS Norm-Standfläche einer Standard-Mikroplatte abweichenden Hauptstandfläche 2, wobei gerade in diesem Moment eine Biopsiekassette 8 von einem Manipulator 23 zumindest im wesentlichen horizontal von der unteren Aufbewahrungseinheit in die obere (von der Hauptstandfläche 2 weg) gestossen wird. Die beiden Aufbewahrungseinheiten 1 stehen auf einer Nebenstandfläche 20, die im wesentlichen rechtwinklig zur Hauptstandfläche 2 ausgerichtet ist. Abweichend von der hier gewählten Darstellung können diese beiden Aufbewahrungseinheiten 1 eine Nebenstandfläche 20 und eine einer der SBS Norm-Standfläche einer Standard-Mikroplatte entsprechende Hauptstandfläche 2 aufweisen.

Der Manipulator 23 ist Teil der Vorrichtung 22 eines Transfersystems 21, mit welchem mindestens zwei Aufbewahrungseinheiten 1 im Register übereinander angeordnet und gegenüber einander verschoben werden können. Die gezeigten Aufbewahrungseinheiten 1 befinden sich in einer gegenseitigen Position, bei welcher nur ein Teil und einander nicht entsprechenden Aufbewahrungskompartimente 3 dieser beiden Aufbewahrungseinheiten 1 genau über einander platziert sind. Zumindest eine dieser beiden Aufbewahrungseinheiten 1 wurde gegenüber der anderen unter Verwendung der Vorrichtung 22 so verschoben, dass jedem beliebigen Aufbewahrungskompartiment 3 der oberen Aufbewahrungseinheit 1 ein beliebiges Aufbewahrungskompartiment 3 der unteren Aufbewahrungseinheit 1 exakt im Register untereinander stehend zugeordnet ist. Ein vorzugsweise robotisierter Manipulator 23 wird nun in ein bestimmtes und individuell ausgewähltes Aufbewahrungskompartiment 3 eingeführt und die sich darin befindende Biopsiekassette 8 in ein vorher zugeordnetes Aufbewahrungskompartiment 3 der anderen Aufbewahrungseinheit 1 (von der Hauptstandfläche 2 weg) gestossen. Der durch den Reibschluss bewirkte Gleitwiderstand zwischen den Haltemitteln 9 und der zu verschiebenden Biopsiekassette 8 bewirkt, dass dieses Biopsiekassette nach dem Übertragen aus der untenliegenden Aufbewahrungseinheit 1 in die obenliegende Aufbewahrungseinheit 1 nicht aus dieser herausfällt. Der Manipulator 23 ist vorzugsweise an die Form der hinteren, nicht abgeschrägten Stirnfläche 14b der Biopsiekassette 8 angepasst und kann eine reibungsverstärkende Beschichtung aufweisen, welche das Abrutschen auf dieser Stirnfläche 14b der Biopsiekassette 8 verhindert.

Wie dargestellt, werden die Biopsiekassetten 8 vorzugsweise hier so in die Aufbewahrungskompartimente 3 einer Aufbewahrungseinheit 1 eingesetzt, dass die vorderen, abgeschrägten Stirnflächen 14a der Biopsiekassetten 8 nach vorne (von der Hauptstandfläche 2 weg) stehen und aus dieser Richtung von Auge oder automatisiert mittels eines entsprechenden Lesegerätes abgelesen werden können. Ein RF-ID Tag wird vorzugsweise im Blindraum 37 an der Unterseite der Biopsiekassette 8 untergebracht (vgl. Fig. 5B).

Alternativ zu der gezeigten Stossrichtung des Manipulators 23 kann diese auch in der Gegenrichtung, also von vorne nach hinten (also von links nach rechts, nicht gezeigt) verlaufen. Von der Darstellung in Fig. 7 abweichend kann die obenliegende Aufbewahrungseinheit einen geschlossenen Boden aufweisen (nicht gezeigt) und lediglich als Sammeleinheit für Biopsiekassetten 8 dienen.

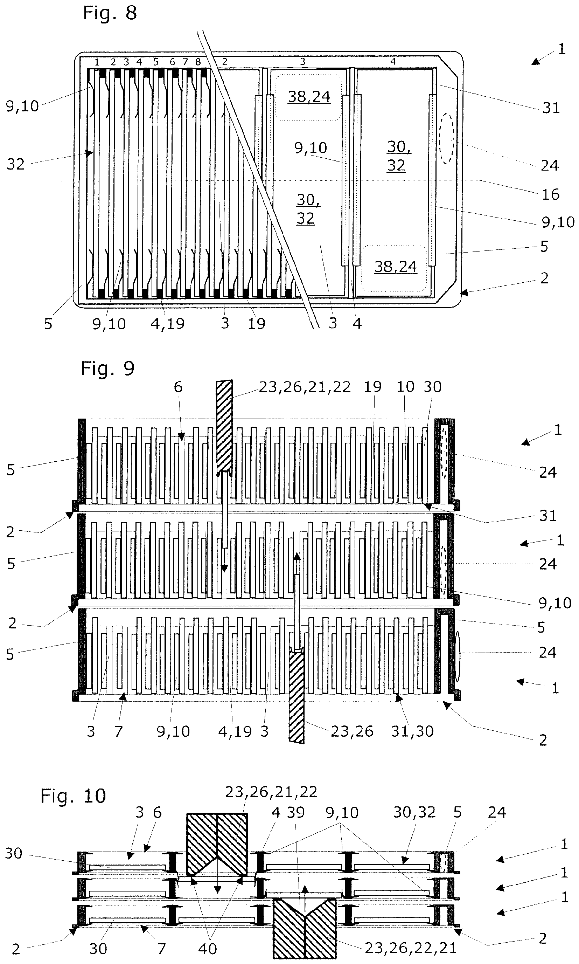

Figur 8 zeigt eine Draufsicht auf eine für Glasobjektträger geeignete Aufbewahrungseinheit 1, in welcher die Glasobjektträger 30 quer und im wesentlichen vertikal stehend, eng gepackt (linke Seite von Fig. 8) oder quer und liegend (Rechte Seite von Fig. 8) angeordnet sind. Bei diesen Anordnungen spielt es grundsätzlich keine Rolle, wie die Oberfläche des Objektträgers zu liegen oder zu stehen kommt, auf der sich die Proben, also beispielsweise die Gewebeschnitte (z.B. aus der Pathologie), Zellausstriche (z.B. Blutausstriche) oder Zellrasen (z.B. aus einer Zellkultur) befinden. In jedem Fall ist dafür gesorgt, dass die Probenregion des Glasobjektträgers 30 mechanisch nicht belastet wird.

Diese Aufbewahrungseinheit 1 für biologische Proben weist eine im wesentlichen horizontale Hauptstandfläche 2 und mehrere Aufbewahrungskompartimente 3 auf, die zumindest teilweise durch Zwischenwände 4 voneinander getrennt und von einem Umfangsrahmen 5 umgeben sind. Dabei sind die Zwischenwände 4 und der Umfangsrahmen 5 im wesentlichen rechtwinklig zur Hauptstandfläche 2 angeordnet. Zudem weisen diese Aufbewahrungskompartimente 3 sowohl eine erste Öffnung 6 als auch eine zweite Öffnung 7 auf. Die Aufbewahrungskompartimente 3 sind an die Form eines Glasobjektträgers 30 angepasst und umfassen Haltemittel 9, welche die durch die erste Öffnung 6 und durch die zweite Öffnung 7 in diese Aufbewahrungskompartimente 3 eingesetzten Glasobjektträger 30 am Herausfallen durch die erste Öffnung 6 und/oder durch die zweite Öffnung 7 hindern. Die Aufbewahrungskompartimente 3 weisen bevorzugt einen im wesentlichen rechteckigen Querschnitt auf und sind entweder zum Aufnehmen von in Bezug auf die Hauptstandfläche 2 je eines auf einer Längskante 31 stehenden oder auf einer Oberfläche 32 liegenden Glasobjektträgers 30 für biologische Proben ausgebildet.

Die Haltemittel 9 sind bevorzugt ausgewählt aus einer Gruppe, die einseitige und doppelseitige Lamellen 10 umfasst. Dieses "einseitig" soll im Zusammenhang mit der vorliegenden Erfindung als "gegen einen anliegenden Objektträger wirkend" verstanden werden. Dieses "doppelseitig" soll im Zusammenhang mit der vorliegenden Erfindung als "gegen zwei anliegende Objektträger wirkend" verstanden werden. Die Haltemittel 9 (hier Lamellen 10) verlaufen entweder im wesentlichen rechtwinklig in Bezug auf die Hauptstandfläche 2 (vgl. linke Seite von Fig. 8) oder im wesentlichen parallel zu der Hauptstandfläche 2 (vgl. rechte Seite von Fig. 8).

Die im wesentlichen rechtwinklig in Bezug auf die Hauptstandfläche 2 angeordneten Lamellen 10 sind zur Ineingriffnahme mit Reibschluss an den Oberflächen 32 eines im wesentlichen vertikal in ein Aufbewahrungskompartiment 3 eingesetzten Glasobjektträgers 30 ausgebildet. Durch diesen Reibschluss werden diese eingesetzten Glasobjektträger 30 am Herausfallen durch die erste Öffnung 6 und/oder durch die zweite Öffnung 7 (egal, auf welche Seite die Aufbewahrungseinheit 1 gekippt wird) gehindert. Diese Lamellen 10 setzen am Umfangsrahmen 5 (nicht gezeigt) oder an sich rechtwinklig in Bezug auf die Hauptstandfläche 2 erstreckenden Führungslamellen 19 an. Diese Führungslamellen 19 übernehmen die Funktion der Zwischenwände 4 und trennen damit zumindest teilweise die Aufbewahrungskompartimenten 3 voneinander. Die Lamellen 10 sind vorzugsweise nur so breit ausgebildet, dass sie die Probenbereiche der Glasobjektträger 30 nicht erreichen. Die Lamellen 10 beaufschlagen die Glasobjektträger 30 und drücken diese federnd gegen die Führungslamellen 19, so dass die Glasobjektträger 30 sicher in ihren Aufbewahrungskompartimenten 3 gehalten sind. Die dichte Packung der Aufbewahrungskompartimente 3 erlaubt das Anordnen von bis zu 24 oder mehr vorzugsweise im wesentlichen vertikal auf einer Längskante 31 stehenden Glasobjektträgern 30 in einer einzigen Aufbewahrungseinheit 1.

Die im wesentlichen parallel zu der Hauptstandfläche 2 angeordneten Lamellen 10 sind zur teilweisen Auflage mit den Oberflächen 32 eines in ein Aufbewahrungskompartiment 3 eingesetzten Glasobjektträgers 30 ausgebildet. Durch diese teilweise Auflage werden diese eingesetzten Glasobjektträger 30 am Herausfallen durch die erste Öffnung 6 und/oder durch die zweite Öffnung 7 (egal, auf welche Seite die Aufbewahrungseinheit 1 gekippt wird) gehindert. Die Lamellen 10 sind hier oben und unten an die Zwischenwände 4 angeformt oder an diesen befestigt (z.B. durch Schweissen oder Kleben, nicht gezeigt) und stehen auf beiden Seiten über diese Zwischenwände 4 hinaus (vgl. Fig. 10). Die am Umfangsrahmen 5 angeordneten oberen und unteren Lamellen 10 befinden bevorzugt auf der gleichen Höhe wie die entsprechenden, an den Zwischenwänden 4 angebrachten Lamellen. Die Lamellen 10 sind jedoch vorzugsweise nur so breit ausgebildet, dass sie die Probenbereiche der Glasobjektträger 30 nicht erreichen. Die Lamellen 10 können an einem Stück ausgebildet sein und sich über eine Teillänge der Aufbewahrungskompartiment 3 erstrecken (vgl. Fig. 8, rechte Seite); sie können aber auch unterteilt sein (nicht gezeigt). Eine alternative Anordnung der Lamellen 10 sieht vor, dass diese an den Querseiten der Aufbewahrungskompartimente 3 angeordnet sind (nicht gezeigt). Kombinationen der gezeigten und alternativen Anordnungen sind ebenfalls vorgesehen. Das liegende Anordnen erlaubt das Aufbewahren von bis zu 4 Glasobjektträgern 30 in einer Aufbewahrungseinheit 1.

Figur 9 zeigt einen Vertikalschnitt durch drei übereinander im Register angeordnete Aufbewahrungseinheiten 1 mit einer der SBS Norm-Standfläche einer Standard-Mikroplatte entsprechenden Hauptstandfläche 2. In diesem Moment wird gerade je ein im wesentlichen vertikal stehender Glasobjektträger 30 von einem ersten Manipulator 23 vertikal von der oberen Aufbewahrungseinheit in die mittlere (gegen die Hauptstandfläche 2 hin) und von einem zweiten Manipulator 23 von der unteren Aufbewahrungseinheit in die mittlere (von der Hauptstandfläche 2 weg) gestossen.

Der Manipulator 23 ist Teil der Vorrichtung 22 eines Transfersystems 21, mit welchem mindestens zwei Aufbewahrungseinheiten 1 im Register übereinander angeordnet und gegenüber einander verschoben werden können. Die gezeigten Aufbewahrungseinheiten 1 befinden sich in einer gegenseitigen Position, bei welcher alle einander entsprechenden Aufbewahrungskompartimente 3 dieser beiden Aufbewahrungseinheiten 1 genau über einander platziert sind. Zumindest eine dieser beiden Aufbewahrungseinheiten 1 kann nun gegenüber der anderen unter Verwendung der Vorrichtung 22 so verschoben werden, dass jedem beliebigen Aufbewahrungskompartiment 3 der oberen Aufbewahrungseinheit 1 ein beliebiges Aufbewahrungskompartiment 3 der unteren Aufbewahrungseinheit 1 exakt im Register untereinander stehend zugeordnet werden kann. Es ist dann ein Leichtes, einen vorzugsweise robotisierten Manipulator 23 in ein bestimmtes und individuell ausgewähltes Aufbewahrungskompartiment 3 einzuführen und den sich darin befindenden Glasobjektträger 30 in ein vorher zugeordnetes Aufbewahrungskompartiment 3 einer zweiten Aufbewahrungseinheit 1 (z.B. gegen die Hauptstandfläche 2 hin) zu stossen. Der durch den Reibschluss bewirkte Gleitwiderstand zwischen den Haltemitteln 9 und dem zu verschiebenden Glasobjektträger 30 bewirkt, dass dieser Glasobjektträger 30 nach dem Übertragen aus der obenliegenden Aufbewahrungseinheit 1 in die untenliegende Aufbewahrungseinheit 1 nicht aus dieser herausfällt. Der Manipulator 23 ist vorzugsweise an die Form des im wesentlichen senkrecht stehenden Glasobjektträgers 30 angepasst und kann zusätzlich eine reibungsverstärkende Beschichtung aufweisen, welche das Abrutschen auf der Längskante 31 des Glasobjektträgers 30 verhindert.

Wie in Figur 9 dargestellt werden die Glasobjektträger 30 vorzugsweise so auf einer Längskante 31 im wesentlichen vertikal stehend in die Aufbewahrungskompartimente 3 einer Aufbewahrungseinheit 1 eingesetzt, dass sie einen (wenn auch minimalen) Abstand voneinander haben. Entlang der oberen Längskante 31 und/oder im diesen Längskanten nahen Bereich der an den Schmalseiten angeordneten Griffbereiche 38 (vgl. auch Fig. 8) der Glasobjektträger 30 sind bevorzugt Identifikationen 24 (z.B. in Form von Barcodemarkierungen) angebracht, so dass diese von Auge oder automatisiert mittels eines entsprechenden Lesegerätes abgelesen werden können. Ein RF-ID Tag 39 wird vorzugsweise in einem Griffbereich 38 des Glasobjektträgers 30 angebracht (vgl. Fig. 8).

Alternativ zu der ersten Stossrichtung des Manipulators 23 kann diese auch in der Gegenrichtung, also von unten nach oben verlaufen. Von der Darstellung in Fig. 9 abweichend können auch nur eine Aufbewahrungseinheit 1 und eine Sammeleinheit eingesetzt werden, wobei die Sammeleinheit unten oder oben liegen kann, auf der von der Aufbewahrungseinheit 1 entfernteren Seite einen geschlossenen Boden aufweist (nicht gezeigt) und lediglich als Sammeleinheit für Glasobjektträger 30 dient. Somit genügt zum Durchführen des alternativen Verfahrens, bei dem ausgewählte Glasobjektträgers 30 von einer Aufbewahrungseinheit 1 oder mehreren solchen Aufbewahrungseinheiten auf eine Sammeleinheit für Glasobjektträger 30 übertragen werden, die Verwendung von mindestens einer Aufbewahrungseinheit 1 für Glasobjektträger 30.

Abweichend von Fig. 9 und entsprechend der in Fig. 7 gezeigten Anordnung können zwei übereinander im Register angeordnete Aufbewahrungseinheiten 1 zusätzlich oder alternativ mit einer von der SBS Norm-Standfläche einer Standard-Mikroplatte abweichenden Hauptstandfläche 2 ausgerüstet sein. Diese alternativen Aufbewahrungseinheiten 1 stehen auf dieser Nebenstandfläche 20, die im wesentlichen rechtwinklig zur Hauptstandfläche 2 ausgerichtet ist. Ein vorzugsweise robotisierter Manipulator 23 wird nun in ein bestimmtes und individuell ausgewähltes Aufbewahrungskompartiment 3 eingeführt und den sich darin befindenden Glasobjektträger 30 in ein vorher zugeordnetes Aufbewahrungskompartiment 3 der anderen Aufbewahrungseinheit 1 (von der Hauptstandfläche 2 weg) stossen. Der durch den Reibschluss bewirkte Gleitwiderstand zwischen den Haltemitteln 9 und dem zu verschiebenden Glasobjektträger 30 bewirkt, dass dieser Glasobjektträger 30 nach dem Übertragen nicht aus dieser anderen Aufbewahrungseinheit 1 herausfällt. Alternativ zu der angedeuteten Stossrichtung des Manipulators 23 kann diese auch in der Gegenrichtung, also von links nach rechts (nicht gezeigt) verlaufen. Ebenfalls kann die obenliegende Aufbewahrungseinheit einen geschlossenen Boden oder Deckel aufweisen (nicht gezeigt) und lediglich als Sammeleinheit für Glasobjektträger 30 dienen.

Figur 10 zeigt einen Vertikalschnitt durch drei übereinander im Register angeordnete Aufbewahrungseinheiten 1 mit einer der SBS Norm-Standfläche einer Standard-Mikroplatte entsprechenden Hauptstandfläche 2. In diesem Moment wird je ein im wesentlichen horizontal liegender Glasobjektträger 30 von einem ersten Manipulator 23 vertikal von der oberen Aufbewahrungseinheit 1 in die mittlere (gegen die Hauptstandfläche 2 hin) und von einem zweiten Manipulator 23 von der unteren Aufbewahrungseinheit 1 vertikal in die mittlere (von der Hauptstandfläche 2 weg) gestossen.

Beim transferierenden Stossen der im wesentlichen horizontal liegenden Objektträger 30 wird dieser gegen die nächstliegenden Lamellen 10 der Aufbewahrungseinheit 1 gedrückt, in welcher der Objektträger 30 anfänglich liegt. Diese Lamellen 10 können diejenigen sein, welche die erste Öffnung 6 des ursprünglichen Aufbewahrungskompartiments 3 einengen. Diese Lamellen 10 können aber auch diejenigen sein, auf welchen der Objektträger 30 liegt und welche die zweite Öffnung 7 des ursprünglichen Aufbewahrungskompartiments 3 einengen. Beim weiteren Bewegen des Objektträgers 30 werden diese vom Objektträger 30 elastisch deformiert, so dass die Lamellen 10 unter Verformung elastisch ausweichen und beispielsweise die beiden Längskanten (vgl. Fig. 9) im Reibschluss temporär beaufschlagen. Gegen diesen Gleitwiderstand wird der Objektträger 30 vom Manipulator 23 weiter gestossen, wobei die das Aufbewahrungskompartiment 3 der nächsten Aufbewahrungseinheit 1 begrenzenden Lamellen 10 ebenfalls elastisch ausweichen und den Objektträger 30 im Reibschluss temporär beaufschlagen. Wird der Objektträger 30 von oben nach unten befördert, so fällt er auf die ihn tragenden Lamellen 10 der neuen Aufbewahrungseinheit 1, sobald er den Einflussbereich der eben verformten Lamellen verlassen hat. Wird der Objektträger 30 von unten nach oben befördert, so wird er beinahe bis zu den das neue Aufbewahrungskompartiment 3 oben einengenden Lamellen 10 weiterbewegt. Der Objektträger 30 verlässt damit den Einflussbereich der eben verformten Lamellen, welche sofort in ihre im wesentlichen vertikale Ursprungslage zurückspringen. Nun kann der Manipulator 23 abgesenkt werden und der von unten nach oben beförderte Objektträger 30 bleibt auf den ihn tragenden Lamellen 10 der neuen Aufbewahrungseinheit 1 liegen.

Aus dieser Beschreibung ergibt sich, dass die transferierten Objektträger 30 zumindest über einen Teil ihres Transferweges von einer Aufbewahrungseinheit 1 zur anderen gegen den Gleitwiderstand von Lamellen 10 bewegt wird. Dieser Gleitwiderstand ermöglicht ein sorgfältiges Ablegen insbesondere eines von oben nach unten gestossenen Objektträgers 30. Der Manipulator 23 weist Ausnehmungen 39 auf, dank welchen die Proben auf dem Objektträger 30 nicht belastet werden. Der Manipulator 23 kann an seinen Stossflächen 40 eine Beschichtung zur Vergrösserung des Gleitwiderstandes of dem Objektträger 30 und/oder zum sanft federnden Beaufschlagen der Objektträger 30 aufweisen.

Ein bevorzugtes Transfersystem zum Transferieren von Biopsiekassetten 8 oder Objektträgern 30 von einer Aufbewahrungseinheit 1 zu einer zweiten Aufbewahrungseinheit 1 oder zu einer Sammeleinheit umfasst bevorzugt eine Vorrichtung 22, mit welcher mindestens zwei Aufbewahrungseinheiten 1 oder mindestens eine Aufbewahrungseinheit 1 und eine Sammeleinheit im Register übereinander angeordnet und gegenüber einander verschoben werden können. Ein solches Transfersystem 21 umfasst vorzugsweise zudem einen Manipulator 23, mit welchem eine in ein individuelles Aufbewahrungskompartiment 3 eingesetzte Biopsiekassette 8 oder ein in ein individuelles Aufbewahrungskompartiment 3 eingesetzter Glasobjektträger 30 von der einen Aufbewahrungseinheit 1 in die andere Aufbewahrungseinheit 1 oder in die Sammeleinheit gestossen werden kann.

Bei einem ersten bevorzugten Transfersystem ist die Vorrichtung 22 zum Anordnen der mindestes zwei Aufbewahrungseinheiten 1 oder mindestens einer Aufbewahrungseinheit 1 und einer Sammeleinheit in übereinanderliegenden, im wesentlichen horizontalen Ebenen ausgebildet. Zudem ist der Manipulator 23 zum im wesentlichen vertikalen Stossen einer Biopsiekassette 8 oder eines Glasobjektträgers 30 gegen die Hauptstandfläche 2 hin oder von dieser weg ausgebildet.

Bei einem zweiten bevorzugten Transfersystem 21 ist die Vorrichtung 22 zum Anordnen der mindestes zwei Aufbewahrungseinheiten 1 oder mindestens einer Aufbewahrungseinheit 1 und einer Sammeleinheit in übereinanderliegenden, im wesentlichen vertikalen Ebenen und dieser Manipulator 23 zum im wesentlichen horizontalen Stossen einer Biopsiekassette 8 oder eines Glasobjektträgers 30 gegen die Hauptstandfläche 2 hin oder von dieser weg ausgebildet.

Solche Transfersysteme 21 umfassen vorzugsweise zudem eine Lagereinrichtung 25 zum Aufbewahren einer Vielzahl von Aufbewahrungseinheiten 1 gemäss einem der Ansprüche 1 bis 15 und einen Roboter 26, mit welchem solche Aufbewahrungseinheiten 1 aus der Lagereinrichtung 25 entnommen und/oder in dieser Lagereinrichtung 25 abgelegt werden können. Dabei sind Lagereinrichtungen 25 speziell bevorzugt, die ein Array von Halterungen 27 mit Auflageflächen 17 umfassen, wobei die Auflageflächen 17 dieser Halterungen 27 zum Beaufschlagen mit der Hauptstandfläche 2 oder mit einer Nebenstandfläche 20 einer Aufbewahrungseinheit 1 ausgebildet sind.

Zum eindeutigen Identifizieren und Verfolgen umfassen die erfindungsgemässen Aufbewahrungseinheiten 1 und die Biopsiekassetten 8 oder Glasobjektträger 30 mindestens eine Identifikation 24, die ausgewählt ist aus RFID-Tags und Barcodes. Die erfindungsgemässen Aufbewahrungseinheiten 1 werden vorzugsweise im Spritzgussverfahren hergestellt und umfassen ein Polymer oder mehrere Polymere, zu denen vorzugsweise Polycarbonat (PC) gehört.

Die Verwendung von in individuellen Aufbewahrungskompartimenten 3 von erfindungsgemässen Aufbewahrungseinheiten 1 für biologische Proben angeordneten Biopsiekassetten 8 oder Glasobjektträgern 30 hat das Aufbewahren und Bereitstellen einer grossen Anzahl solcher biologischen Proben zum Zweck. Dabei umfasst jede Aufbewahrungseinheit 1 eine Hauptstandfläche 2 und mehrere Aufbewahrungskompartimente 3, die zumindest teilweise durch Zwischenwände 4 voneinander getrennt und von einem Umfangsrahmen 5 umgeben sind. Zudem sind die Zwischenwände 4 und der Umfangsrahmen 5 im wesentlichen rechtwinklig zur Hauptstandfläche 2 angeordnet und diese Aufbewahrungskompartimente 3 weisen sowohl eine erste Öffnung 6 als auch eine zweite Öffnung 7 auf. Die erfindungsgemässen Aufbewahrungskompartimente 3 sind an die Form von Biopsiekassetten 8 oder an die Form von Glasobjektträgern 30 angepasst. Diese Verwendung sieht vor, dass je eine Biopsiekassette 8 oder je ein Glasobjektträger 30 durch die erste Öffnung 6 oder durch die zweite Öffnung 7 in ein solches Aufbewahrungskompartiment 3 eingesetzt wird, das Haltemittel 9 umfasst, welche die eingesetzte Biopsiekassette 8 oder den eingesetzten Glasobjektträger 30 am Herausfallen durch die erste Öffnung 6 und durch die zweite Öffnung 7 hindern.

Zur robotisierten Handhabung und Lagerung dieser Aufbewahrungseinheiten 1 weisen diese bevorzugt eine Hauptstandfläche 2 auf, die der SBS Norm-Standfläche einer Standard-Mikroplatte zumindest angenähert ist. Eine oder mehrere dieser Biopsiekassetten 8 oder Glasobjektträger 30 können robotisiert und automatisiert aus den entsprechenden Aufbewahrungskompartimenten 3 einer ersten Aufbewahrungseinheit 1 entfernt und in ausgewählte Aufbewahrungskompartimente 3 einer zweiten Aufbewahrungseinheit 1 oder einer Sammeleinheit eingesetzt werden. Vorzugsweise werden zwei diese Aufbewahrungseinheiten 1 mit einer Vorrichtung 22 eines Transfersystems 21 im Register übereinander angeordnet und gegenüber einander verschoben werden, wobei mit einem Manipulator 23 dieses Transfersystems 21 Biopsiekassetten 8 oder Glasobjektträger 30 vertikal von der oberen Aufbewahrungseinheit 1 in die darunter liegende Aufbewahrungseinheit 1 oder Sammeleinheit gestossen werden.

Kombinationen und Variationen der gezeigten bzw. beschriebenen Lamellen 10, Kissen 11 und Führungslamellen 19 gehören zum Umfang der vorliegenden Erfindung.

### Bezugszeichenliste:

- 1: Aufbewahrungseinheit
- 2: Hauptstandfläche
- 3: Aufbewahrungskompartimente
- 4: Zwischenwände
- 5: Umfangsrahmen
- 6: erste Öffnung
- 7: zweite Öffnung
- 8: Biopsiekassette
- 9: Haltemittel
- 10: Lamellen
- 11: Kissen
- 12: Lagerflächen
- 13: Höhenflächen
- 14: Stirnfläche (14a,14b)
- 15: Querachse
- 16: Längsachse
- 17: Auflageflächen
- 18: Mittelwand
- 19: Führungslamellen
- 20: Nebenstandfläche

- 21: System
- 22: Vorrichtung
- 23: Manipulator
- 24: Identifikation
- 25: Lagereinrichtung
- 26: Roboter
- 27: Deckel
- 28: Korb
- 29: Scharnier
- 30: Glasobjektträger
- 31: Längskante
- 32: Oberfläche
- 33: Schlitze
- 34: Lasche
- 35: Zapfen
- 36: Boden
- 37: Blindraum
- 38: Griffbereiche
- 39: Ausnehmungen
- 40: Stossflächen

## Patentansprüche

1. Aufbewahrungseinheit (1) für biologische Proben, mit einer horizontalen Hauptstandfläche (2) und mehreren Aufbewahrungskompartimenten (3), die zumindest teilweise durch Zwischenwände (4) voneinander getrennt und von einem Umfangsrahmen (5) umgeben sind, wobei die Zwischenwände (4) und der Umfangsrahmen (5) Teil der Aufbewahrungseinheit (1) und rechtwinklig zur Hauptstandfläche (2) angeordnet sind, und wobei die Aufbewahrungskompartimente (3) sowohl eine erste Öffnung (6) als auch eine zweite Öffnung (7) aufweisen, wobei die Aufbewahrungskompartimente (3) Haltemittel (9) umfassen und je eine Biopsiekassette (8) oder je ein Glasobjektträger (30) gegen einen Gleitwiderstand der Haltemittel (9) durch die erste Öffnung (6) und durch die zweite Öffnung (7)
in ein solches Aufbewahrungskompartiment (3) einsetzbar ist, **dadurch gekennzeichnet, dass** die Aufbewahrungskompartimente (3) an die Form einer Biopsiekassette (8) oder an die Form eines Glasobjektträgers (30) angepasst sind und zumindest eines der Aufbewahrungskompartimente (3) eine solche Biopsiekassette (8) oder einen solchen Glasobjektträger (30) umfasst und die Haltemittel (9) dazu ausgebildet sind, die vertikal stehenden Biopsiekassetten (8) oder Glasobjektträger (30) am Herausfallen durch die erste Öffnung (6) und am Herausfallen durch die zweite Öffnung (7) zu hindern, wobei die Haltemittel (9) so ausgebildet sind, dass die erste Öffnung (6) und die zweite Öffnung (7) offengehalten werden.

2. Aufbewahrungseinheit (1) nach Anspruch 1, **dadurch gekennzeichnet, dass** die Aufbewahrungskompartimente (3) einen rechteckigen Querschnitt aufweisen und zum Aufnehmen von in Bezug auf die Hauptstandfläche (2) je einer auf einer Stirnfläche (14) stehenden Biopsiekassette (8) oder je einem auf einer Längskante (31) stehenden Glasobjektträgers (30) für biologische Proben ausgebildet sind.

3. Aufbewahrungseinheit (1) nach Anspruch 1, **dadurch gekennzeichnet, dass** die Aufbewahrungskompartimente (3) einen rechteckigen Querschnitt aufweisen und zum Aufnehmen von in Bezug auf die Hauptstandfläche (2) je einer auf einer Lagerfläche (12) stehenden Biopsiekassette (8) oder je einem auf einer Längskante (31) stehenden Glasobjektträgers (30) für biologische Proben ausgebildet sind.

4. Aufbewahrungseinheit (1) nach einem der vorhergehenden Ansprüche, **dadurch gekennzeichnet, dass** die Haltemittel (9) ausgewählt sind aus einer Gruppe, die einseitige und doppelseitige Lamellen (10) sowie einseitige und doppelseitige Kissen (11) umfasst.

5. Aufbewahrungseinheit (1) nach Anspruch 4, **dadurch gekennzeichnet, dass** sie rechtwinklig in Bezug auf die Hauptstandfläche (2) verlaufende Lamellen (10) umfasst, die zur Ineingriffnahme mit Reibschluss an den Lagerflächen (12) oder an den Höhenflächen (13) einer in ein Aufbewahrungskompartiment (3) eingesetzten Biopsiekassette (8) ausgebildet sind.

6. Aufbewahrungseinheit (1) nach Anspruch 4, **dadurch gekennzeichnet, dass** sie rechtwinklig in Bezug auf die Hauptstandfläche (2) verlaufende Lamellen (10) umfasst, die zur Ineingriffnahme mit Reibschluss an Oberflächen (32) eines in ein Aufbewahrungskompartiment (3) eingesetzten Glasobjektträgers (30) ausgebildet sind.

7. Aufbewahrungseinheit (1) nach Anspruch 4, **dadurch gekennzeichnet, dass** sie parallel in Bezug auf die Hauptstandfläche (2) verlaufende Lamellen (10) umfasst, die zur teilweisen Auflage für Oberflächen (32) eines in ein Aufbewahrungskompartiment (3) eingesetzten Glasobjektträgers (30) ausgebildet sind.

8. Aufbewahrungseinheit (1) nach Anspruch 4, **dadurch gekennzeichnet, dass** sie Kissen (11) umfasst, die zur Ineingriffnahme mit Reibschluss an den Lagerflächen (12) oder an den Höhenflächen (13) einer in ein Aufbewahrungskompartiment (3) eingesetzten Biopsiekassette (8) ausgebildet sind.

9. Aufbewahrungseinheit (1) nach Anspruch 8, **dadurch gekennzeichnet, dass** die Kissen (11) sich mit gleichem Wirkungsquerschnitt über die gesamte Höhe des Aufbewahrungskompartiments (3) erstrecken.

10. Aufbewahrungseinheit (1) nach einem der Ansprüche 8 oder 9, **dadurch gekennzeichnet, dass** die Kissen (11) einseitig oder doppelseitig ausgebildet und in einer Reihe Aufbewahrungskompartimente (3) auf einer mittig und quer zu diesen Aufbewahrungskompartimenten (3) verlaufenden Längsachse (16) angeordnet sind.

11. Aufbewahrungseinheit (1) nach einem der Ansprüche 8 oder 9, **dadurch gekennzeichnet, dass** die Kissen (11) einseitig oder doppelseitig ausgebildet und in einer Reihe Aufbewahrungskompartimente (3) auf einer mittig und längs zu diesen Aufbewahrungskompartimenten (3) verlaufenden Querachse (15) angeordnet sind.

12. Aufbewahrungseinheit (1) nach einem der Ansprüche 10 oder 11, **dadurch gekennzeichnet, dass** die doppelseitigen Kissen (11) einer Reihe Aufbewahrungskompartimente (3) auf zwei quer zu den Aufbewahrungskompartimenten (3) verlaufenden Längsachsen (16) angeordnet sind.

13. Aufbewahrungseinheit (1) nach einem der vorhergehenden Ansprüche, **dadurch gekennzeichnet, dass** sie eine Mittelwand (18) umfasst, die rechtwinklig in Bezug auf die Hauptstandfläche (2) verlaufende Führungslamellen (19) umfasst, welche in die beidseits der Mittelwand (18) angeordneten Aufbewahrungskompartimente (3) abstehen.

14. Aufbewahrungseinheit (1) nach einem der vorhergehenden Ansprüche, **dadurch gekennzeichnet, dass** ihre Hauptstandfläche (2) der SBS Norm-Standfläche einer Standard-Mikroplatte entspricht.

15. Aufbewahrungseinheit (1) nach einem der vorhergehenden Ansprüche, **dadurch gekennzeichnet, dass** sie zumindest eine Nebenstandfläche (20) umfasst, welche zusammen mit der Hauptstandfläche (2) einen Winkel von zumindest annähernd 90 ° einschliesst.

16. Transfersystem (21), welches eine Vorrichtung (22) umfasst, mit welcher mindestens zwei Aufbewahrungseinheiten (1) nach einem der vorhergehenden Ansprüche oder mindestens eine Aufbewahrungseinheit (1) nach einem der vorhergehenden Ansprüche und eine Sammeleinheit im Register übereinander angeordnet und gegenüber einander verschoben werden können, **dadurch gekennzeichnet, dass** das Transfersystem (21) zudem einen Manipulator (23) umfasst, mit welchem eine in ein individuelles Aufbewahrungskompartiment (3) eingesetzte Biopsiekassette (8) oder ein in ein individuelles Aufbewahrungskompartiment (3) eingesetzter Glasobjektträger (30) von einer Aufbewahrungseinheit (1) in die andere Aufbewahrungseinheit (1) oder in die Sammeleinheit gestoßen werden kann.

17. Transfersystem (21) nach Anspruch 16, **dadurch gekennzeichnet, dass** die Vorrichtung (22) zum Anordnen der mindestens zwei Aufbewahrungseinheiten (1) oder mindestens einer Aufbewahrungseinheit (1) und einer Sammeleinheit in übereinanderliegenden, horizontalen Ebenen ausgebildet ist und der Manipulator (23) zum vertikalen Stoßen einer Biopsiekassette (8) oder eines Glasobjektträgers (30) zu der Hauptstandfläche (2) hin oder von dieser weg ausgebildet ist.

18. Transfersystem (21) nach Anspruch 16, **dadurch gekennzeichnet, dass** die Vorrichtung (22) zum Anordnen der mindestens zwei Aufbewahrungseinheiten (1) oder mindestens einer Aufbewahrungseinheit (1) und einer Sammeleinheit in übereinanderliegenden, vertikalen Ebenen ausgebildet ist und der Manipulator (23) zum horizontalen Stoßen einer Biopsiekassette (8) oder eines Glasobjektträgers (30) zu der Hauptstandfläche (2) hin oder von dieser weg ausgebildet ist.

19. Transfersystem (21) nach einem der Ansprüche 16 bis 18 mit einer Vorrichtung (22), **dadurch gekennzeichnet, dass** es eine Lagereinrichtung (25) zum Aufbewahren einer Vielzahl von Aufbewahrungseinheiten (1) nach einem der Ansprüche 1 bis 15 und einen Roboter (26) umfasst, mit welchem solche Aufbewahrungseinheiten (1) aus der Lagereinrichtung (25) entnommen und/oder in der Lagereinrichtung (25) abgelegt werden können.

20. Transfersystern (21) nach Anspruch 19, **dadurch gekennzeichnet, dass** die Lagereinrichtung (25) ein Array von Halterungen (27) mit Auflageflächen (17) umfasst, wobei die Auflageflächen (17) dieser Halterungen zum Beaufschlagen mit der Hauptstandfläche (2) oder mit einer Nebenstandfläche (20) einer Aufbewahrungseinheit (1) ausgebildet sind.

21. Transfersystem (21) nach einem der Ansprüche 16 bis 20, **dadurch gekennzeichnet, dass** die Aufbewahrungseinheiten (1) und die Biopsiekassetten (8) oder Glasobjektträger (30) mindestens eine Identifikation (24) umfassen, die ausgewählt ist aus RFID-Tags und Barcodes.

22. Verwendung von in individuellen Aufbewahrungskompartimenten (3) von Aufbewahrungseinheiten (1) für biologische Proben angeordneten Biopsiekassetten (8) oder Glasobjektträgern (30) zum Aufbewahren und Bereitstellen einer großen Anzahl solcher biologischen Proben, wobei jede Aufbewahrungseinheit (1) eine horizontale Hauptstandfläche (2) und mehrere Aufbewahrungskompartimente (3) umfasst, die zumindest teilweise durch Zwischenwände (4) voneinander getrennt und von einem Umfangsrahmen (5) umgeben sind, wobei die Zwischenwände (4) und der Umfangsrahmen (5) Teil der Aufbewahrungseinheit (1) und rechtwinklig zur Hauptstandfläche (2) angeordnet sind, und wobei die Aufbewahrungskompartimente (3) sowohl eine erste Öffnung (6) als auch eine zweite Öffnung (7) aufweisen, wobei die Aufbewahrungskompartimente (3) Haltemittel (9) umfassen und je eine Biopsiekassette (8) oder je ein Glasobjektträger (30) gegen einen Gleitwiderstand der Haltemittel (9) durch die erste Öffnung (6) oder durch die zweite Öffnung (7) in ein solches Aufbewahrungskompartiment (3) in einer vertikal stehenden Position eingesetzt wird, **dadurch gekennzeichnet, dass** die Aufbewahrungskompartimente (3) an die Form von Biopsiekassetten (8) oder an die Form von Glasobjektträgern (30) angepasst sind und zumindest eines der Aufbewahrungskompartimente (3) eine solche Biopsiekassette (8) oder einen solchen Glasobjektträger (30) umfasst und die Haltemittel (9) die eingesetzten Biopsiekassetten (8) oder die eingesetzten Glasobjektträger (30) in ihrer vertikal stehenden Lagerposition am Herausfallen durch die erste Öffnung (6) und am Herausfallen durch die zweite Öffnung (7) hindern, wobei die Haltemittel (9) so ausgebildet sind, dass die erste Öffnung (6) und die zweite Öffnung (7) offengehalten werden.

23. Verwendung nach Anspruch 22, wobei die Aufbewahrungseinheiten (1) eine SBS Norm-Standfläche einer Standard-Mikroplatte aufweisen, **dadurch gekennzeichnet, dass** eine oder mehrere dieser Biopsiekassetten (8) oder Glasobjektträger (30) robotisiert aus den entsprechenden Aufbewahrungskompartimenten (3) einer ersten Aufbewahrungseinheit (1) entfernt und in ausgewählte Aufbewahrungskompartimente (3) einer zweiten Aufbewahrungseinheit (1) oder einer Sammeleinheit eingesetzt werden.

24. Verwendung nach Anspruch 23, **dadurch gekennzeichnet, dass** zwei diese Aufbewahrungseinheiten (1) mit einer Vorrichtung (22) eines Transfersystems (21) im Register übereinander angeordnet und gegenüber einander verschoben werden, wobei mit einem Manipulator (23) des Transfersystems (21) Biopsiekassetten (8) oder Glasobjektträger (30) vertikal von der oberen Aufbewahrungseinheit (1) in die darunter liegende Aufbewahrungseinheit (1) oder Sammeleinheit gestoßen werden.

## Claims

1. A storage unit (1) for biological samples, with a horizontal main footprint (2) and a plurality of storage compartments (3), which are at least partially separated from one another by partitions (4) and are enclosed by a peripheral frame (5), wherein the partitions (4) and the peripheral frame (5) are part of the storage unit (1) and are disposed perpendicular to the main footprint (2), and wherein the storage compartments (3) have both a first opening (6) as well as a second opening (7), wherein the storage compartments (3) comprise retention means (9) and a biopsy cassette (8) or a glass slide (30) can be inserted into each compartment against a sliding resistance of the retention means (9) through the first opening (6) and through the second opening (7) into such a storage compartment (3), **characterized in that** the storage compartments (3) are tailored to the shape of a biopsy cassette (8) or to the shape of a glass slide (30) and at least one of the storage compartments (3) comprises such a biopsy cassette (8) or such a glass slide (30) and the retention means (9) are configured in a manner so as to prevent the vertically standing biopsy cassettes (8) or glass slides (30) from falling out through the first opening (6) and from falling out through the second opening (7), wherein the retention means (9) are configured in a manner such that the first opening (6) and the second opening (7) are kept open.

2. The storage unit (1) as claimed in claim 1, **characterized in that** the storage compartments (3) have a rectangular cross-section and are each configured to receive, with respect to the main footprint (2), one biopsy cassette (8) for biological samples standing on a front face (14) or one glass slide (30) for biological samples standing on a longitudinal edge (31).

3. The storage unit (1) as claimed in claim 1, **characterized in that** the storage compartments (3) have a rectangular cross-section and are each configured to receive, with respect to the main footprint (2), one biopsy cassette (8) for biological samples standing on a support surface (12) or one glass slide for biological samples (30) standing on a longitudinal edge (31).

4. The storage unit (1) according to one of the preceding claims, **characterized in that** the retention means (9) are selected from a group which comprises single-sided and double-sided lamellae (10) as well as single-sided and double-sided cushions (11).

5. The storage unit (1) as claimed in claim 4, **characterized in that** it comprises lamellae (10) running perpendicular to the main footprint (2) which are configured to engage with a friction lock on the support surfaces (12) or on the vertical surfaces (13) of a biopsy cassette (8) inserted into a storage compartment (3) .

6. The storage unit (1) as claimed in claim 4, **characterized in that** it comprises lamellae (10) running perpendicular to the main footprint (2) which are configured to engage with a friction lock on surfaces (32) of a glass slide (30) inserted into a storage compartment (3).

7. The storage unit (1) as claimed in claim 4, **characterized in that** it comprises lamellae (10) running parallel to the main footprint (2) which are configured to support portions of surfaces (32) of a glass slide (30) inserted into a storage compartment (3) .

8. The storage unit (1) as claimed in claim 4, **characterized in that** it comprises cushions (11) which are configured to engage with a friction lock on the support surfaces (12) or on the vertical surfaces (13) of a biopsy cassette (8) inserted into a storage compartment (3).

9. The storage unit (1) as claimed in claim 8, **characterized in that** the cushions (11) extend over the entire height of the storage compartments (3) with an identical effective cross-section.

10. The storage unit (1) as claimed in claim 8 or claim 9, **characterized in that** the cushions (11) are single-sided or double-sided in configuration and are disposed in a row of storage compartments (3) on a longitudinal axis (16) running centrally and transversely to these storage compartments (3).

11. The storage unit (1) as claimed in claim 8 or claim 9, **characterized in that** the cushions (11) are single-sided or double-sided in configuration and are disposed in a row of storage compartments (3) on a transverse axis (15) running centrally and longitudinally to these storage compartments (3).

12. The storage unit (1) as claimed in claim 10 or claim 11, **characterized in that** the double-sided cushions (11) of a row of storage compartments (3) are disposed on two longitudinal axes (16) running transversely to the storage compartments (3).

13. The storage unit (1) according to one of the preceding claims, **characterized in that** it comprises a central wall (18) which comprises guide lamellae (19) running perpendicular to the main footprint (2), which project into the storage compartments (3) disposed on both sides of the central wall (18).

14. The storage unit (1) according to one of the preceding claims, **characterized in that** its main footprint (2) corresponds to the SBS standard footprint of a standard microplate.

15. The storage unit (1) according to one of the preceding claims, **characterized in that** it comprises at least one secondary footprint (20) which encloses an angle of at least approximately 90° with the main footprint (2).

16. A transfer system (21) which comprises a device (22) with which at least two storage units (1) as claimed in one of the preceding claims or at least one storage unit (1) as claimed in one of the preceding claims and a collection unit can be disposed one above the other in register and be displaced with respect to one another, **characterized in that** the transfer system (21) additionally comprises a manipulator (23) with which a biopsy cassette (8) inserted into an individual storage compartment (3) or a glass slide (30) inserted into an individual storage compartment (3) can be pushed from one storage unit (1) into the other storage unit (1) or into the collection unit.

17. The transfer system (21) as claimed in claim 16, **characterized in that** the device (22) is configured in order to dispose the at least two storage units (1) or at least one storage unit (1) and a collection unit in horizontal planes lying one above the other, and the manipulator (23) is configured for pushing a biopsy cassette (8) or a glass slide (30) vertically towards the main footprint (2) or away from it.

18. The transfer system (21) as claimed in claim 16, **characterized in that** the device (22) is configured for disposing the at least two storage units (1) or at least one storage unit (1) and a collection unit in vertical planes lying one above the other, and the manipulator (23) is configured for pushing a biopsy cassette (8) or a glass slide (30) horizontally towards the main footprint (2) or away from it.

19. The transfer system (21) as claimed in one of claims 16 to 18 with a device (22), **characterized in that** it comprises a warehouse unit (25) for storing a plurality of storage units (1) as claimed in one of claims 1 to 15 and a robot (26), with which such storage units (1) can be removed from the warehouse unit (25) and/or deposited in the warehouse unit (25).

20. The transfer system (21) as claimed in claim 19, **characterized in that** the storage unit (25) comprises an array of retainers (27) with bearing surfaces (17), wherein the bearing surfaces (17) of these retainers are configured to impinge upon the main footprint (2) or a secondary footprint (20) of a storage unit (1).

21. The transfer system (21) as claimed in one of claims 16 to 20, **characterized in that** the storage unit (1) and the biopsy cassettes (8) or glass slides (30) comprise at least one identifier (24) which is selected from RFID tags and barcodes.

22. Use of biopsy cassettes (8) or glass slides (30) disposed in individual storage compartments (3) of storage units (1) for biological samples, for storing and providing a large number of such biological samples, wherein each storage unit (1) comprises a horizontal main footprint (2) and a plurality of storage compartments (3) which are at least partially separated from one another by partitions (4) and are enclosed by a peripheral frame (5), wherein the partitions (4) and the peripheral frame (5) are part of the storage unit (1) and are disposed perpendicular to the main footprint (2), and wherein the storage compartments (3) have both a first opening (6) as well as a second opening (7), wherein the storage compartments (3) comprise retention means (9) and a biopsy cassette (8) or a glass slide (30) can be inserted into each such storage compartment (3) in a vertical standing position, through the first opening (6) and through the second opening (7) against a sliding resistance of the retention means (9), **characterized in that** the storage compartments (3) are tailored to the shape of biopsy cassettes (8) or to the shape of glass slides (30) and at least one of the storage compartments (3) comprises such a biopsy cassette (8) or such a glass slide (30) and in its vertical standing storage position, the retention means (9) prevent the inserted biopsy cassette (8) or the inserted glass slide (30) from falling through the first opening (6) and/or from falling through the second opening (7), wherein the retention means (9) are configured in a manner such that the first opening (6) and the second opening (7) are kept open.

23. Use as claimed in claim 22, wherein the storage units (1) have an SBS standard footprint for a standard microplate, **characterized in that** one or more of these biopsy cassettes (8) or glass slides (30) is/are robotically removed from the corresponding storage compartments (3) of a first storage unit (1) and inserted into selected storage compartments (3) of a second storage unit (1) or a collection unit.

24. The use as claimed in claim 23, **characterized in that** two of these storage units (1) are disposed one above the other in register and displaced with respect to one another with a device (22) of a transfer system (21), wherein biopsy cassettes (8) or glass slides (30) are pushed vertically from the upper storage unit (1) into the storage unit (1) or collection unit lying underneath with a manipulator (23) of the transfer system (21).

## Revendications

1. Unité de stockage (1) pour des échantillons biologiques, avec une face d'appui principale (2) horizontale et plusieurs compartiments de stockage (3), qui sont séparés les uns des autres au moins en partie par des cloisons intermédiaires (4) et sont entourés par un cadre périphérique (5), dans laquelle les cloisons intermédiaires (4) et le cadre périphérique (5) font partie de l'unité de stockage (1) et sont disposés à angle droit par rapport à la face d'appui principale (2), et dans laquelle les compartiments de stockage (3) présentent à la fois une première ouverture (6) et une deuxième ouverture (7), dans laquelle les compartiments de stockage (3) comprennent des moyens de maintien (9) et respectivement une cassette à biopsies (8) ou respectivement un porte-objet en verre (30) peuvent être insérés dans un tel compartiment de stockage (3) par la première ouverture (6) et par la deuxième ouverture (7) à l'encontre d'une résistance au glissement des moyens de maintien (9), **caractérisée en ce que** les compartiments de stockage (3) sont adaptés à la forme d'une cassette à biopsies (8) ou à la forme d'un porte-objet en verre (30) et au moins un des compartiments de stockage (3) comprend une cassette à biopsies (8) de ce type ou un porte-objet en verre (30) de ce type et les moyens de maintien (9) sont réalisés pour empêcher les cassettes à biopsies (8) ou les supports d'objet en verre (30) situés à la verticale de tomber par la première ouverture (6) et de tomber par la deuxième ouverture (7), dans laquelle les moyens de maintien (9) sont réalisés de telle sorte que la première ouverture (6) et la deuxième ouverture (7) sont maintenues ouvertes.

2. Unité de stockage (1) selon la revendication 1, **caractérisée en ce que** les compartiments de stockage (3) présentent une section transversale rectangulaire et sont réalisés pour loger, par rapport à la face d'appui principale (2), respectivement une cassette à biopsies (8) située sur une face frontale (14) ou respectivement un porte-objet en verre (30) situé sur une arête longitudinale (31) pour des échantillons biologiques.

3. Unité de stockage (1) selon la revendication 1, **caractérisée en ce que** les compartiments de stockage (3) présentent une section transversale rectangulaire et sont réalisés pour loger, par rapport à la face d'appui principale (2), respectivement une cassette à biopsies (8) située sur une face de montage (12) ou respectivement un porte-objet en verre (30) situé sur une arête longitudinale (31) pour des échantillons biologiques.

4. Unité de stockage (1) selon l'une quelconque des revendications précédentes, **caractérisée en ce que** les moyens de maintien (9) sont choisis parmi un groupe, qui comprend des lamelles (10) à un côté ou à double côté ainsi que des coussins (11) à un côté ou à double côté.

5. Unité de stockage (1) selon la revendication 4, **caractérisée en ce qu'**elle comprend des lamelles (10) s'étendant à angle droit par rapport à la face d'appui principale (2), qui sont réalisées pour venir en prise par friction sur les faces de montage (12) ou sur les faces en hauteur (13) d'une cassette à biopsies (8) insérée dans un compartiment de stockage (3).

6. Unité de stockage (1) selon la revendication 4, **caractérisée en ce qu'**elle comprend des lamelles (10) s'étendant à angle droit par rapport à la face d'appui principale (2), qui sont réalisées pour venir en prise par friction sur des surfaces (32) d'un porte-objet en verre (30) inséré dans un compartiment de stockage (3).

7. Unité de stockage (1) selon la revendication 4, **caractérisée en ce qu'**elle comprend des lamelles (10) s'étendant de manière parallèle par rapport à la face d'appui principale (2), qui sont réalisées pour la réception partielle pour des surfaces (32) d'un porte-objet en verre (30) inséré dans un compartiment de stockage (3) .

8. Unité de stockage (1) selon la revendication 4, **caractérisée en ce qu'**elle comprend des coussins (11), qui sont réalisés pour venir en prise par friction sur les faces de montage (12) ou sur les faces en hauteur (13) d'une cassette à biopsies (8) insérée dans un compartiment de stockage (3).

9. Unité de stockage (1) selon la revendication 8, **caractérisée en ce que** les coussins (11) s'étendent avec une section transversale efficace identique sur la totalité de la hauteur du compartiment de stockage (3).

10. Unité de stockage (1) selon l'une quelconque des revendications 8 ou 9, **caractérisée en ce que** les coussins (11) sont réalisés avec un seul côté ou un double côté et sont disposés dans une rangée de compartiments de stockage (3) sur un axe longitudinal (16) s'étendant au centre et de manière transversale par rapport auxdits compartiments de stockage (3).

11. Unité de stockage (1) selon l'une quelconque des revendications 8 ou 9, **caractérisée en ce que** les coussins (11) sont réalisés avec un seul côté ou un double côté et sont disposés dans une rangée de compartiments de stockage (3) sur un axe transversal (15) s'étendant au centre et de manière longitudinale par rapport auxdits compartiments de stockage (3).

12. Unité de stockage (1) selon l'une quelconque des revendications 10 ou 11, **caractérisée en ce que** les coussins (11) à double côté d'une rangée de compartiments de stockage (3) sont disposés sur deux axes longitudinaux (16) s'étendant de manière transversale par rapport aux compartiments de stockage (3).

13. Unité de stockage (1) selon l'une quelconque des revendications précédentes, **caractérisée en ce qu'**elle comprend une cloison centrale (18), qui comprend des lamelles de guidage (19) s'étendant à angle droit par rapport à la face d'appui principale (2), lesquelles dépassent dans les compartiments de stockage (3) disposés de part et d'autre de la cloison centrale (18).

14. Unité de stockage (1) selon l'une quelconque des revendications précédentes, **caractérisée en ce que** sa face d'appui principale (2) correspond à la face d'appui de norme SBS d'une microplaque standard.

15. Unité de stockage (1) selon l'une quelconque des revendications précédentes, **caractérisée en ce qu'**elle comprend au moins une face d'appui secondaire (20), laquelle forme conjointement avec la face d'appui principale (2) un angle d'au moins approximativement 90°.

16. Système de transfert (21), lequel comprend un dispositif (22), avec lequel au moins deux unités de stockage (1) selon l'une quelconque des revendications précédentes ou au moins une unité de stockage (1) selon l'une quelconque des revendications précédentes et une unité de collecte peuvent être disposées les unes au-dessus des autres ou coulissées les unes à l'encontre des autres dans le registre, **caractérisé en ce que** le système de transfert (21) comprend de plus un manipulateur (23) avec lequel une cassette à biopsies (8) insérée dans un compartiment de stockage (3) individuel ou un porte-objet en verre (30) inséré dans un compartiment de stockage (3) individuel peuvent être heurtés par une unité de stockage (1) dans l'autre unité de stockage (1) ou dans l'unité de collecte.

17. Système de transfert (21) selon la revendication 16, **caractérisé en ce que** le dispositif (22) est réalisé pour disposer les au moins deux unités de stockage (1) ou au moins une unité de stockage (1) et une unité de collecte dans des plans horizontaux situés les uns au-dessus des autres et le manipulateur (23) est réalisé pour heurter verticalement une cassette à biopsies (8) ou un porte-objet en verre (30) en direction de la face d'appui principale (2) ou de manière à s'en éloigner.

18. Système de transfert (21) selon la revendication 16, **caractérisé en ce que** le dispositif (22) est réalisé pour disposer les au moins deux unités de stockage (1) ou au moins une unité de stockage (1) et une unité de collecte dans des plans verticaux situés les uns au-dessus des autres et le manipulateur (23) est réalisé pour heurter horizontalement une cassette à biopsies (8) ou un porte-objet en verre (30) en direction d'une face d'appui principale (2) ou de manière à s'en éloigner.

19. Système de transfert (21) selon l'une quelconque des revendications 16 à 18 avec un dispositif (22), **caractérisé en ce qu'**il comprend un équipement de montage (25) pour stocker une pluralité d'unités de stockage (1) selon l'une quelconque des revendications 1 à 15 et un robot (26), avec lequel des unités de stockage (1) de ce type peuvent être retirées hors de l'équipement de montage (25) et/ou peuvent être déposées dans l'équipement de montage (25).

20. Système de transfert (21) selon la revendication 19, **caractérisé en ce que** l'équipement de montage (25) comprend un réseau de supports (27) avec des faces de réception (17), dans lequel les faces de réception (17) desdits moyens de maintien sont réalisées pour être soumises à l'action de la face d'appui principale (2) ou à l'action d'une face d'appui secondaire (20) d'une unité de stockage (1).

21. Système de transfert (21) selon l'une quelconque des revendications 16 à 20, **caractérisé en ce que** les unités de stockage (1) et les cassettes à biopsies (8) ou les supports d'objet en verre (30) comprennent au moins une identification (24) qui est choisie parmi les étiquettes RFID et les codes à barres.

22. Utilisation de cassettes à biopsies (8) ou de supports d'objet en verre (30) disposés dans des compartiments de stockage (3) individuels d'unités de stockage (1) pour des échantillons biologiques pour stocker et pour fournir un grand nombre d'échantillons biologiques de ce type, dans laquelle chaque unité de stockage (1) comprend une face d'appui principale (2) horizontale et plusieurs compartiments de stockage (3), qui sont séparés les uns des autres au moins en partie par des cloisons intermédiaires (4) et sont entourés par un cadre périphérique (5), dans laquelle les cloisons intermédiaires (4) et le cadre périphérique (5) font partie de l'unité de stockage (1) et sont disposés à angle droit par rapport à la face d'appui principale (2), et dans laquelle les compartiments de stockage (3) présentent à la fois une première ouverture (6) et une deuxième ouverture (7), dans laquelle les compartiments de stockage (3) comprennent des moyens de maintien (9) et respectivement une cassette à biopsies (8) et respectivement un porte-objet en verre (30) sont insérés dans un compartiment de stockage (3) de ce type dans une position située à la verticale par la première ouverture (6) ou par la deuxième ouverture (7) à l'encontre d'une résistance de glissement des moyens de maintien (9), **caractérisée en ce que** les compartiments de stockage (3) sont adaptés à la forme de cassettes à biopsies (8) ou à la forme de porte-objets en verre (30) et au moins un des compartiments de stockage (3) comprend une cassette à biopsies (8) de ce type ou un porte-objet en verre (30) de ce type et les moyens de maintien (9) empêchent les cassettes à biopsies (8) insérées ou les porte-objets en verre (30) insérés dans leur position de montage située à la verticale de tomber par la première ouverture (6) et de tomber par la deuxième ouverture (7), dans laquelle les moyens de maintien (9) sont réalisés de telle sorte que la première ouverture (6) et la deuxième ouverture (7) sont maintenues ouvertes.

23. Utilisation selon la revendication 22, dans laquelle les unités de stockage (1) présentent une face d'appui de norme SBS d'une microplaque standard, **caractérisée en ce qu'**une ou plusieurs desdites cassettes à biopsies (8) ou un ou plusieurs desdits porte-objets en verre (30) sont enlevés de manière robotisée hors des compartiments de stockage (3) correspondants d'une première unité de stockage (1) et sont insérés dans des compartiments de stockage (3) choisis d'une deuxième unité de stockage (1) ou d'une unité de collecte.

24. Utilisation selon la revendication 23, **caractérisée en ce que** deux desdites unités de stockage (1) sont disposées avec un dispositif (22) d'un système de transfert (21) dans le registre l'une au-dessus de l'autre et sont coulissées l'une en vis-à-vis de l'autre, dans laquelle des cassettes à biopsies (8) ou des porte-objets en verre (30) sont heurtés verticalement par l'unité de stockage (1) supérieure dans l'unité de stockage (1) ou unité de collecte située en dessous par un manipulateur (23) du système de transfert (21).
